# EUROPEAN PATENT SPECIFICATION

(11) **EP 1 553 924 B1**
(45) Date of publication and mention of the grant of the patent: **08.12.2010**
(21) Application number: 03761520.0
(22) Date of filing: 26.06.2003
(51) Int. Cl.: A61K 9/127, A61K 31/335, A61K 51/12, A61P 35/00

(54) **NOVEL METHOD OF STABILIZING DIAGNOSTIC AND THERAPEUTIC COMPOUNDS IN A CATIONIC CARRIER SYSTEM**
NEUES VERFAHREN ZUR STABILISIERUNG VON DIAGNOSTISCHEN UND THERAPEUTISCHEN VERBINDUNGEN IN EINEM KATIONISCHEN TRÄGERSYSTEM
PROCEDE POUR STABILISER DES COMPOSES DESTINES AU DIAGNOSTIC OU A LA THERAPIE DANS UN SYSTEME SUPPORT CATIONIQUE

(30) Priority: 26.06.2002 US 391244 P; 26.06.2002 US 391245 P; 26.06.2002 US 391246 P; 21.08.2002 EP 02018724; 23.08.2002 EP 02018907; 04.03.2003 EP 03004744
(43) Date of publication of application: 20.07.2005
(73) Proprietor: MediGene AG, 82152 Planegg/Martinsried (DE)
(72) Inventor: HAAS, Heinrich, 80337 München (DE); FICHERT, Thomas, 66606 St. Wendel (DE); SCHULZE, Brita, 82432 Walchensee (DE); PEYMANN, Toralf, 30625 Hannover (DE); MICHAELIS, Uwe, 82362 Weilheim (DE); TEIFEL, Michael, 64331 Weiterstadt (DE)
(74) Representative: Weiss, Wolfgang
(86) International application number: PCT/EP2003/006765
(87) International publication number: WO 2004/002455

(56) References cited:
- WO-A-01/17508
- WO-A-01/82899
- WO-A-97/10234
- DE-A- 19 604 484
- US-A- 4 970 074
- CAMPBELL R B ET AL: "INFLUENCE OF CATIONIC LIPIDS ON THE STABILITY AND MEMBRANE PROPERTIES OF PACLITAXEL-CONTAINING LIPOSOMES" JOURNAL OF PHARMACEUTICAL SCIENCES, AMERICAN PHARMACEUTICAL ASSOCIATION. WASHINGTON, US, vol. 90, no. 8, August 2001 (2001-08), pages 1091-1105, XP001101828 ISSN: 0022-3549
- EMERSON D L: "Liposomal delivery of camptothecins" PHARMACEUTICAL SCIENCE AND TECHNOLOGY TODAY, ELSEVIER TRENDS JOURNALS, CAMBRIDGE,, GB, vol. 3, no. 6, 1 June 2000 (2000-06-01), pages 205-209, XP002235822 ISSN: 1461-5347

## Description

The present invention relates to a method of stabilizing a low molecular weight compound in a cationic liposome, wherein said compound has a low solubility in a lipid membrane and/or a low permeability across a lipid membrane. Preferably, the compound is modified in order to increase lipid membrane solubility and/or lipid membrane permeability. After delivery of the cationic liposome to a target site, particularly a target site in an organism, the modification may be reversed and the low molecular weight compound may enact its desired activity.

Generally, water-soluble molecules of low molecular weight can be formulated with liposomes by encapsulation into the aqueous compartment of the latter. To this end, a liposomal suspension is produced in the aqueous solution of the compound, for example by reconstitution of a thin lipid film at the inner wall of a flask with the aqueous solution of the compound or by the well known ethanol injection method. The amount of the encapsulated drug corresponds to the initial concentration of the component in the aqueous solution multiplied by the ratio between the encapsulated and the free volume. The amount of the encapsulated drug is usually rather small, due to the unfavourable ratio between the inner aqueous volume of the liposomes and the total aqueous volume of the suspension is small. In order to separate the non-encapsulated fraction of the compound dialysis, diafiltration or similar methods are performed. Unfortunately, during this separation step the overwhelming part of the encapsulated compound is lost.

Highly lipophilic molecules can be formulated with liposomes by embedding these molecules into the liposomal membrane. Unfortunately, some of these molecules can be embedded only to a rather low extent. The hydrophobic compound is thought to be 'dissolved' in the hydrocarbon region of the bilayer membrane, and only a limited number of molecules can be inserted in that slab. A very prominent example for those molecules is Paclitaxel, an anti-cancer drug, which allows membrane loading with a final molar ratio between lipid and drug of only up to 33:1 (3 mol%).

Emerson D.L. (PSST Vol. 3, No. 6 June 2000) discloses the liposomal delivery of camptothecins (CPTs) such as topotecan, irinotecan, lurtotecan or SN38. Liposomes consisted of electroneutral dimyristoylphosphatidylcholine (DMPS) or negatively charged dimyristoylphosphatidylglycerol (DMPG) and were small unilamellar vesicles. The embedded CPTs thereby were up to 100 % in its lactone form, which is the biologically active form. However, uncharged or negatively charged liposomes do not specifically target to a particular tissue so that severe side effects are expected to occur if a human patient is treated with a therapeutically effective dose of these liposomes.

Summarizing these disadvantages, the problem underlying the present invention was to provide a method of producing a stable formulation of a low molecular weight compound, which may be water-soluble or lipophilic, and which by itself may be poorly soluble in a lipid membrane and/or which has a low membrane permeability across said membrane, for an efficient delivery of said compound to a target site.

The problem was solved by providing a method of stabilizing a low molecular weight compound from 200 to 5000 Da in a liposome, wherein said compound is poorly soluble in a lipid membrane and/or has a low permeability across said membrane, comprising the steps of
(a) providing said compound comprising modifying said compound with a moiety that has a negative net charge, wherein said compound is modified to have a negative net charge,
   a) associating the compound of step a) with a cationic amphiphile having a positive net charge and optionally at least one further amphiphile anionic and/or neutral amphiphile having a negative and/or neutral net charge and
   b) forming a cationic liposome having a positive zeta potential.

In this method, the compound having a negative net charge is preferably provided in a solution, e.g. an aqueous solution, and is selectively bound to a cationic lipid via electrostatic and optionally amphiphilic forces. By means of this association, stable liposomes comprising the compound can be formed. In that case, a high fraction of the compound present in the solution can be bound to the cationic lipid and thus incorporated into to the liposome, and therefore, only a low fraction is lost.

The low molecular weight compound can be a diagnostic agent with a negative net charge, a therapeutic agent with a negative net charge or a combination thereof. The negative net charge can be due to the presence of one or several anionic groups such as carboxylate, sulfate, sulfonate, phosphate, nitrate, or combinations thereof. These groups may be introduced via modification, e.g. via chemical derivatization. Preferably, the compound is an organic molecule or contain an organic moiety.

The low molecular weight compound may have a molar weight of up to 5000 Da, preferably 200 to 5000 Da. Before modification the compound is preferably characterized by a very high lipophilicity as e.g. indicated by a octanol/water distribution coefficient (logD) higher than about 3, preferably higher than about 4. Further, the compound needs to be feasible for modification/derivatization, e.g. by having a reactive functional group.

A diagnostic agent can be selected from diagnostic or imaging agents such as dyes, near-infrared dyes, fluorescent dyes, gold particles, iron oxide particles and other contrast agents including paramagnetic molecules, X-ray attenuating compounds (for CT and X-ray) contrast agents for ultrasound, X-ray and y-ray emitting isotopes (scintigraphy), and positron-emitting isotopes (PET). Specific examples are selected from iodinated aromatic compounds such as lopamidol, ^{99m}Tc- DTPA (diethylene thiamine penta-acetic acid) or ¹¹¹In-DTPA and derivatives thereof, fluorescent compounds such as rhodamine or fluoresceine, ferrite particles or Gd complexes such as Gd-DTPA or Gd-DOTA.

A therapeutic agent can be selected from drugs such as an anti-inflammatory drug, an anti-cancer drug, an enzymatic drug, an antibiotic substance, an antioxidant, a hormone drug, an angiogenesis inhibiting agent, a smooth muscle cell-proliferation/migration inhibitor, a platelet aggregation inhibitor, a release inhibitor for a chemical mediator, and a proliferation/migration inhibitor for vascular endothelium. Specific examples are selected from taxanes, from other agents interacting with microtubuli such as epothilones, discodermolide, laulimalide, isolaulimalide, eleutherobin, colchicines and derivatives thereof, vinca alkaloids such as vinorelbine, from platinum complexes such as oxaliplatin, from camptothecins, from anthracyclines such as doxorubicin or from statins (e.g., lovastatin).

In the present invention the concentration of the low molecular weight imaging agent in the liposome highly depends on the selected class of imaging agent i.e. on the desired application. Whereas radioactive nuclei will be loaded in very low concentrations in the nM range, MRI and X-ray contrast agents can be loaded in concentrations of 1 mM to 1000 mM. Liposomal dyes will be loaded in a concentration range of 0.1 mM to 100 mM, while gold nanoparticles are in the range of several µM. For a PET imaging experiments of a human a fluorine-18 dose of 10 mCi is required.

The therapeutic compound may be loaded into the liposomal membrane in a therapeutically effective concentration, e.g. in a concentration range of 0.1 mol% to 50 mol%, preferably of 1 mol% to 20 mol%, preferably of 3 mol% to 15 mol%, even more preferably of 5 mol% to 10 mol% based on the liposomal components.

The cationic amphiphile used in the present invention is an amphiphile with a positive net charge. It can be monovalent or polyvalent. It may be selected from lipids, lysolipids or pegylated lipids with a positive net charge. Useful cationic lipids thereby include:
DDAB, dimethyldioctadecyl ammonium bromide; N-[1-(2,3-dioleoyloxy) propyl]-N,N,N-trimethyl ammonium methylsulfate (DOTAP); 1,2-diacyloxy-3-trimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleoyloxy)propyl]-N,N-dimethyl amine (DODAP); 1,2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N,N-trimethylammonium chloride (DOTMA); 1,2-diaikyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS);, 3β-[N-(N',N'-dimethylaminoethane)carbamoyl]cholesterol (DC-Chol); 2,3-dioleoyloxy-N-(2-(sperminecarboxamido)-ethyl)-N,N-dimethyl-1-propanaminium trifluoro-acetate (DOSPA); β-alanyl cholesterol; cetyl trimethyl ammonium bromide (CTAB); diC14-amidine; N-tert-butyl-N'-tetradecyl-3-tetradecylaminopropionamidine; 14Dea2; N-(alphatrimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl(alkenyl)-3-(2-hydroxyethyl)-imidazolinium chloride derivatives as described by Solodin et al. (1995) Biochem. 43:13537-13544, such as 1-[2-(9(Z)-octadecenoyloxy) ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl) imidazolinium chloride (DPTIM), 2,3-dialkyloxypropyl quaternary ammonium compound derivatives, contain a hydroxyalkyl moiety on the quaternary amine, as described e.g. by Felgner et al. [Felgner et al. J. Biol. Chem. 1994, 269, 2550-2561] such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. [US5498633]. In a preferred embodiment the cationic amphiphile is selected from a quaternary ammonium salt such as N-[1-(2,3-diacyloxy)propyl]-N,N,N-trimethyl ammonium, wherein a pharmaceutically acceptable counter anion of the quaternary amino compound is selected from the group consisting of chloride, bromide, fluoride, iodide, nitrate, sulfate, methyl sulfate, phosphate, acetate, benzoate, citrate, glutamate or lactate.

Preferred liposomes of the present invention comprise DOTAP, DODAP, analogues of DOTAP or DODAP or any other cationic lipid in an amount of at least 20+x mol% of the total liposome-forming lipid, preferably of at least 30+x mol%, more preferably of at least 40+x mol% and further DOPC, pegylated lipids or any other lipid that has a neutral net charge in an amount of up to 80-2x mol%, preferably up to 70-2x mol% and most preferably up to 60-2x mol% where x mol% is the amount of the loaded compound and the amount of an optionally present neutrally or negatively charged amphiphile.

The cationic liposomes of the present invention comprise at least an amount of 30 mol% cationic lipids, preferably 40 mol%, more preferably 50 mol%, even more preferred 60 mol%, 70 mol%, 80 mol%, or up to 99.9 mol% and are characterized by having a positive zeta potential in about 0.05 M KCl solution at pH 7.5 at room temperature.

Anionic and/or neutral amphiphiles are characterized by a neutral or negative net charge and can be selected from sterols or lipids such as cholesterol, phospholipids, lysolipids, lysophospholipids, sphingolipids or pegylated lipids with a negative or neutral net change. Useful anionic and neutral lipids thereby include: Phosphatidic acid, phosphatidylserine, phosphatidylglycerol, phosphatidylinositol (not limited to a specific sugar), fatty acids, sterols containing a carboxylic acid group, cholesterol, 1,2-diacyl-sn-glycero-3-phosphoethanolamine, including but not limited to dioleoyl (DOPE), 1,2-diacyl-glycero-3-phosphocholin and sphingomyelin. The fatty acids linked to the glycerol backbone are not limited to a specific length or number of double bonds. Phospholipids may also have two different fatty acids. In a preferred embodiment the neutral amphiphile is diacylphosphatidylcholine.

In step c) of the inventive method the liposome can be formed by a lipid film or by an infusion procedure. The lipid film procedure thereby comprises the steps of
a) providing
   i. a lipid film comprising a cationic amphiphile and optionally at least one anionic and/or neutral amphiphile and said compound and
   ii. an aqueous solution or
b) providing
   i. a lipid film comprising a cationic amphiphile and optionally at least one anionic and/or neutral amphiphile and
   ii. an aqueous solution comprising said compound and
c) suspending said lipid film in said aqueous solution.

The infusion procedure comprises the steps of
a) adding an organic solution comprising a cationic amphiphile and optionally at least one anionic and/or neutral amphiphile to an aqueous solution of said compound or
b) adding an organic solution comprising a cationic amphiphile and optionally at least one anionic and/or neutral amphiphile and said compound to an aqueous solution.

The organic solution used in one of the production procedures comprises an organic solvent selected from methanol, ethanol, propanol, isopropanol, ethylene glycol, tetrahydrofuran, chloroform, tert-butanol, diethylether or mixtures of these solvents. The aqueous solution preferably comprises a stabilizing agent such as a cryoprotectant which is selected from a sugar or an alcohol or a combination thereof such as trehalose, maltose, sucrose, glucose, lactose, dextran, mannitol or sorbitol and used in the range of up to 20 % (m/v). Preferably the stabilizing agent is used in the range of 1% (m/v) to 20 % (m/v) and most preferably in the range of 5 % (m/v) to 10 % (m/v) with respect of the total volume of the dispersion of the liposome.

Once the liposome is formed, at least one dialysis and/or at least one homogenisation and/or optionally at least one sterile filtration and/or optionally a freeze-drying and/or reconstitution step may be carried out subsequently.

The cationic liposome obtainable by the method as described above is preferably characterized by having an overall positive charge which refers to an excess of positively charged molecules in the outer molecular layer. The liposome can comprise an excess of cationic lipids of at least 20 mol%, preferably at least 30 mol% and most preferably at least 40 mol%. As an example, if the liposome contains 10 mol% negatively charged lipids or negatively charged modified compound the amount of positively charged lipid has to be at least 30 %, preferably at least 40 mol% and most preferably at least 50 mol% in order to fulfil the charge requirements.

The cationic liposome is stabilized via electrostatic forces and optionally amphiphilic interactions and preferably characterized by at least one of the following features:
- a zeta potential in the range of 25 mV to 100 mV in 0.05 mM KCl solution at pH 7.5 at room temperature, preferably in the range of 35 mV to 70 mV in 0.05 mM KCl solution at pH 7.5 at room temperature,
- it is virtually free of the unmodified compound (if a modification of the compound is required to provide a negative net charge),
- it comprises 1 to 50 mol % of the compound, preferably 5 to 20 mol % of said compound,
- it comprises 30 to less than 100 mol % of the cationic amphiphile, preferably 50 to 90 mol % of the cationic amphiphile,
   is composed of particles having a particle diameter ranging from 5 nm to 5 µm, preferably from 25 nm to 500 nm and more preferably from 100 nm to 300 nm.

All technical and scientific terms used in this specification shall have the same meaning as commonly understood by persons of ordinary skill in the art to which the present invention pertains.

"About" in the context of amount values refers to an average deviation of maximum +/- 20 %, preferably +/- 10 % based on the indicated value. For example, an amount of 30 mol% cationic lipid refers to 30 mol% +/- 6 mol% and preferably 30 mol% +/- 3 mol% cationic lipid with respect to the total lipid/amphiphile molarity.

"Active ingredient" refers to an agent that is diagnostically or therapeutically effective.

"Amphiphile" refers to a molecule consisting of a water-soluble (hydrophilic) and an oil-soluble (lipophilic) part. The lipophilic part preferably contains at least one alkyl group having at least 10, particularly at least 12 carbon atoms.

"Associating" a negatively charged compound to a cationic amphiphile means binding of said compound to the cationic organic molecule by electrostatic and/or amphiphilic forces. The net charge of the molecular aggregation of the said compound and the cationic amphiphile is thought to be close to zero. The aggregation is preferably characterized by a cationic/anionic charge ratio close to 1:1, but also charge ratios of 1.5:1 or >2:1 may be used depending on the nature of the molecules.

"Camptothecin drug" refers to camptothecin or a derivative thereof. A camptothecin derivative is obtained from any chemical derivatization of camptothecin. In the sketch of the molecule, the most frequent derivatization sites are outlined as R₁-R₅. In the table, typical examples for derivatization at the different sites are listed. Any combination of these examples and any other derivatization may be performed. The compound may be present as a hydrochloride. The lactone ring may be seven-membered instead of six-membered.

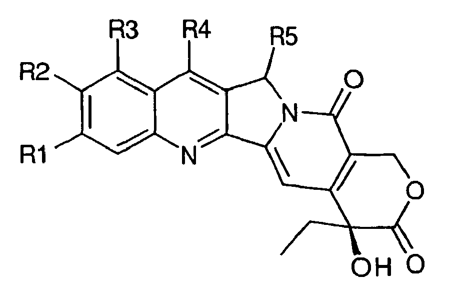

| Name | R1 | R2 | R3 | R4 | R5 |
|---|---|---|---|---|---|
| Camptothecin | H | H | H | H | H |
| 9-Nitro-camptothecin | H | H | NO₂ | H | H |
| 9-Amino-camptothecin | H | H | NH₂ | H | H |
| 10-Hydroxy-camptothecin | H | OH | H | H | H |
| Topotecan | H | OH | N-(CH₃)₂ | H | H |
| SN38 | H | OH | H | CH₂-CH₃ | H |
| Camptosar^{®} | H | 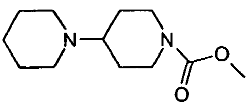 | H | CH₂-CH₃ | H |
| Lurtotecan^{®} | R1 and R2 is: 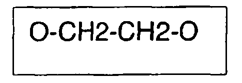 | | H | H | H |
| DX-8951f | F | CH₃ | R₃ and R₄ is: -CH2-CH2-CH (NH₂)- | | H |

"Cancer" refers to the more common forms of cancers such as bladder cancer, breast cancer, colorectal cancer, endometrial cancer, head and neck cancer, leukaemia, lung cancer, lymphoma, melanoma, non-small-cell lung cancer, ovarian cancer, prostate cancer and to childhood cancers such as brain stem glioma, cerebellar astrocytoma, cerebral astrocytoma, ependymoma, Ewing's sarcoma/family of tumors, germ cell tumor, extracranial, hodgkin's disease, leukemia, acute lymphoblastic, leukemia, acute myeloid, liver cancer, medulloblastoma, neuroblastoma, non-hodgkin's lymphoma, osteosarcoma/malignant fibrous histiocytoma of bone, retinoblastoma, rhabdomyosarcoma, soft tissue sarcoma, supratentorial primitive neuroectodermal and pineal tumors, unusual childhood cancers, visual pathway and hypothalamic glioma, Wilms' Tumor and other childhood kidney tumors and to less common cancers including acute lymphocytic leukaemia, adult acute myeloid leukaemia, adult non-hodgkin's lymphoma, brain tumor, cervical cancer, childhood cancers, childhood sarcoma, chronic lymphocytic leukaemia, chronic myeloid leukaemia, esophageal cancer, hairy cell leukaemia, kidney cancer, liver cancer, multiple myeloma, neuroblastoma, oral cancer, pancreatic cancer, primary central nervous system lymphoma, skin cancer, small-cell lung cancer.

"Carrier" refers to a diluent, adjuvant, excipient, or vehicle with which a diagnostic or therapeutic agent is administered. The term also refers to a pharmaceutically acceptable component(s) that contains, complexes or is otherwise associated with an active ingredient to facilitate the transport of such an agent to its intended target site. Carriers include those known in the art, such as liposomes, polymers, lipid complexes, serum albumin, antibodies, cyclodextrins and dextrans, chelates, and other supramolecular assemblies.

"Cationic" refers to an agent that has a net positive charge or positive zeta potential at physiological pH.

The term "cationic lipid" is used herein to encompass any lipid of the invention (as enumerated above) that is cationic. The lipid will be determined as being cationic when the lipid has a positive charge at physiological pH. Where there are fatty acids present on the cationic lipid, they could be 12-24 carbons in length, containing up to 6 unsaturations (double bonds), and linked to the backbone by either acyl or ether linkages; there could also only be one fatty acid chain linked to the backbone. Where there is more than one fatty acid linked to the backbone, the fatty acids could be different (asymmetric). Mixed formulations are also possible.

Cationic liposomes are prepared from the cationic lipids themselves, or in admixture with other lipids, particularly neutral lipids such as cholesterol; 1,2-diacyl-sn-glycero-3-phosphoethanolamines (including but not limited to dioleoyl (DOPE); 1,2-diacyl-sn-glycero-3-phosphocholines; natural egg yolk phosphatidyl choline (PC), and the like; synthetic mono-and diacylphosphoethanolamines. Asymmetric fatty acids, both synthetic and natural, and mixed formulations, for the above diacyl derivatives may also be included.

"Colloids" or "colloidal particles" refer to particles dispersed in a medium in which they are insoluble and have a size between 10 nm and 5000 nm.

"Cryoprotectant" refers to a substance that helps to protect a species from the effect of freezing.

"Derivative" refers a compound derived from some other compound while maintaining its general structural features.

"Diagnostic agent" refers to a pharmaceutically acceptable agent that can be used to localize or visualize a target region by various methods of detection. Diagnostic or imaging agents include those known in the art, such as dyes, fluorescent dyes, gold particles, iron oxide particles and other contrast agents including paramagnetic molecules, X-ray attenuating compounds (for CT and X-ray) contrast agents for ultrasound, X-ray emitting isotopes (scintigraphy), and positron-emitting isotopes (PET).

"Diagnostically effective" refers to an agent that is effective to localize or identify a target site for monitoring or imaging purposes.

"Disease characterized by enhanced angiogenic activity" refers to processes such as wound healing, tissue inflammation, arthritis, asthma, tumor growth, and diabetic retinopathy.

"Drug" as used herein refers to a pharmaceutically acceptable pharmacologically active substance, physiologically active substances and/or substances for diagnosis use.

"Homogenisation" refers to a physical process that achieves a uniform distribution between several components. One example is high-pressure homogenisation.

The term "lipid" is used in its conventional sense as a generic term encompassing fats, lipids, alcohol-ethersoluble constitutents of protoplasm, which are insoluble in water. Lipids may be fats, fatty oils, essential oils, waxes, steroid, sterols, phospholipids, glycolipids, sulpholipids, aminolipids, chromolipids, and fatty acids. The term encompasses both naturally occurring and synthetic lipids. Preferred lipids in connection with the present invention are: steroids and sterol, particularly cholesterol, phospholipids, including phosphatidyl and phosphatidylcholines and phosphatidylethanolamines, and sphingomyelins. Where there are fatty acids, they could be about 12-24 carbon chains in length, containing up to 6 double bonds, and linked to the backbone, the fatty acids could be different (asymmetric), or there may be only 1 fatty acid chain present, e.g., lysolecithins. Mixed formulations are also possible, particularly when the non-cationic lipids are derived from natural sources, such as lecithins (phosphatidylcholines) purified from egg yolk, bovine heart, brain, or liver, or soybean.

"Liposome" refers to a microscopic spherical membrane-enclosed vesicle (about 50-2000 nm diameter). The term "liposome" encompasses any compartment enclosed by a lipid bilayer. Liposomes are also referred to as lipid vesicles. In order to form a liposome the lipid molecules comprise elongated nonpolar (hydrophobic) portions and polar (hydrophilic) portions. The hydrophobic and hydrophilic portions of the molecule are preferably positioned at two ends of an elongated molecular structure. When such lipids are dispersed in water they spontaneously form bilayer membranes referred to as lamellae. The lamellae are composed of two mono layer sheets of lipid molecules with their non-polar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell. Thus, the bilayer of a liposome has similarities to a cell membrane without the protein components present in a cell membrane. As used in connection with the present invention, the term liposome includes multilamellar liposomes, which generally have a diameter in the range of about 1 to 10 micrometers and are comprised of anywhere from two to hundreds of concentric lipid bilayers alternating with layers of an aqueous phase, and also includes unilamellar vesicles which are comprised of a single lipid layer and generally have a diameter in the range of about 20 to about 400 nanometers (nm), about 50 to about 300 nm, about 300 to about 400 nm, about 100 to about 200 nm, which vesicles can be produced by subjecting multilamellar liposomes to ultrasound, by extrusion under pressure through membranes having pores of defined size, or by high pressure homogenization.

Preferred liposomes would be unilamellar vesicles, which have a single lipid bilayer, and a diameter in the range of 25-400nm.

"Low molecular weight compound" refers to a molecule with a molecular weight less than 5000 Daltons.

"Lysolipid" refers to a lipid where one fatty acid ester has been cleaved resulting in a glycerol backbone bearing one free hydroxyl group.

"Lysophospholipid" refers to a phospholipid where one fatty acid ester has been cleaved resulting in a glycerol backbone bearing one free hydroxyl group.

Mol percent (or mol%) defines a fraction of a concentration. Mol percent e.g. refers to the ratio (given in percent) of moles of a lipid or a lipid-soluble species to its total lipid content (given in moles) in 1 liter. One liter of a liposomal formulation comprising 5 mmol of DOTAP, 4.7 mmol of DOPC, and 0.3 mmol of paclitaxel is 10 mM with respect to its total lipid content. In this case, DOTAP contributes 50 mol percent to the liposomal formulation, DOPC 47 and paclitaxel 3 mol percent. This formulation can be described as 10 mM DOTAP/DOPC/Paclitaxel 50/47/3.

"Micelle" refers to an aggregate of molecules in a colloid.

"Modifying" refers to making partial changes of the chemical structure of a compound.

"Negatively charged lipids" refers to lipids that have a negative net charge such as phosphatidic acid, phosphatidylserine, phosphatidylglycerol, phosphatidylinositol (not limited to a specific sugar), fatty acids, sterols containing a carboxylic acid group. The fatty acids linked to the glycerol backbone are not limited to a specific length or number of double bonds.

"Neutral lipids" refers to lipids that have a neutral net charge such as cholesterol, 1,2-diacyl-sn-glycero-3-phosphoethanolamine, including but not limited to dioleoyl (DOPE, a large family of derivatives is available form Avanti Polar Lipids), 1,2-diacyl-glycero-3-phosphocholines (a large family of derivatives is available form Avanti Polar Lipids), Sphingomyelin.

"Particle diameter" refers to the size of a particle measured by dynamic light scattering (DLS) employing a Malvern Zetasizer 3000.

"Pegylated lipid" refers to a lipid bearing one ore more polyethylene glycol residues.

"Pharmaceutical composition" refers to a combination of two or more different materials with superior pharmaceutical properties than are possessed by either component.

"Phospholipid" refers to a lipid having both a phosphate group and one or more fatty acids.

"Reversible" refers to any chemical process that can be made to change in such a way that the process is reversed.

"Sterol" refers to a steroid alcohol. Steroids are derived from the compound called cyclopentanoperhydrophenanthrene. Well-known examples of sterols include cholesterol, lanosterol, and phytosterol.

"Taxane drug" as used herein refers to the class of antineoplastic agents having a mechanism of microtubule action and having a structure that includes the unusual taxane ring structure and a stereospecific side chain that is required for cytostatic activity.

"Taxanes" refers to an anti-angiogenic agent. Also included within the term "taxane" are a variety of known derivatives, including both hydrophilic derivatives, and hydrophobic derivatives. Taxane derivatives include, but not limited to, galactose and mannose derivatives described in International Patent Application No. WO 99/18113; piperazino and other derivatives described in WO 99/14209; taxane derivatives described in WO99/09021, WO 98/22451, and U. S. Patent No. 5,869,680; 6-thio derivatives described in WO 98/28288; sulfenamide derivatives described in U. S. Patent No. 5,821,263; and taxol derivative described in U. S. Patent No. 5,415,869.

"Paclitaxel" (which should be understood herein to include analogues, formulations, and derivatives such as, for example, docetaxel, taxotere (a formulation of docetaxel), 10-desacetyl analogs of paclitaxel and 3'N-desbenzoyl-3'N-t-butoxycarbonyl analogs of paclitaxel) may be readily prepared utilizing techniques known to those skilled in the art (see also WO 94/07882, WO 94/07881, WO 94/07880, WO 94/07876, WO 93/23555, WO 93/10076; U. S. Pat. Nos. 5,294,637; 5,283,253; 5,279,949; 5,274,137; 5,202,448; 5,200,534; 5,229,529; and EP 590,267), or obtained from a variety of commercial sources, including for example, Sigma Chemical Co., St. Louis, Mo. (T7402 from Taxus brevifolia; or T-1912 from Taxus yannanensis). Paclitaxel should be understood to refer to not only the common chemically available form of paclitaxel, but analogs (e. g., taxotere, as noted above) and paclitaxel conjugates (e. g., paclitaxel-PEG, paclitaxeldextran,or paclitaxel-xylose).

"Therapeutic agent" refers to a species that reduces the extent of the pathology of a disease such as cancer. Such a compound may, for example, reduce primary tumor growth and, preferably, the metastatic potential of a cancer. Alternatively, such a compound may reduce tumor vascularity, for example either by decreasing microvessel size or number or by decreasing the blood vessel density ratio.

"Virtually free" or "substantially free" of a species is defined as not detectable by HPTLC.

"Zeta potential" refers to a surface potential of a particle such as a colloidal particle measured with an instrument such as a Zetasizer 3000 using Laser Doppler micro-electrophoresis under the conditions specificed. The zeta potential describes the potential at the boundary between bulk solution and the region of hydrodynamic shear or diffuse layer.

The advantages of the present invention can be described as follows: The loaded compound is chemically stabilized in the inventive liposome. Stabilizing effects are due to complexing with the positively charged amphiphile and optionally due to modification of the compound (negatively charged and amphiphilic derivative). Both steps are critical for the observation that the inventive formulations are virtually free from degradation products and free from the unmodified compound, which does not have the charge and amphiphilic property requirement.

The amphiphilic characteristics allow high loading of the compound for most efficient drug delivery. This is due to the improved stabilizing interactions (amphiphilic interactions) between the disclosed composition and other amphiphiles. However, single chain amphiphiles, such as stearyl amine, are not suitable at higher concentrations than 10 mol% since they significantly lower the physical stability of the liposomal formulations. Thus, cationic non-single chain amphiphiles (lipids) are used in the inventive method.

The overall favourable physicochemical properties of the formulation are responsible for more efficient delivery of the loaded compound to the target site in vivo. The advantage results in improved treatment, such as minimized side effects after systemic administration. Furthermore, drug resistance after treatment is not observable. This is due to the uptake of the drug by the target cells which significantly differs from treatment with other drug formulations.

As outlined in the following, it is proposed to modify the molecular properties of the compound in a way, that the interaction with the cationic lipid is strengthened. This can be achieved by attaching a negative charge to the molecule and/or by making it more amphipatic. The compound then binds tightly to the lipid and may be solubilized within lipid bilayers/membranes as an integral component of the latter.

Modifying the compound therein comprises
a) covalently linking a negatively charged moiety to said compound, e. g. by an ester, thioester, ether, thioether, amide, amine, carbon-carbon bond or a Schiff Base,
b) chelating said compound by a negatively charged ligand or
c) encarcerating said compound within a negatively charged moiety such as a carcerand, calixarene, fullerene, crown or anti-crown ether.

Preferably, the modification is a reversible modification, i.e. the modification may be reversed at a desired target site, e.g. a target site in an organism, whereby the low molecular weight compound is released in an active form.

The negatively charged moiety can be selected from molecules having at least one, preferably more than one, acidic group such as carboxylic acids, e.g. selected from lactic acid, citric acid, succinic acid, glutaric acid, maleic acid, fumaric acid, malonic acid, adipic acid or glutamic acid. The negatively charged moiety is not limited to carboxylic acids, also other acids containing a heteroatom such as sulfur or phosphorus or others can be used. Negatively charged moieties include also polymers of the latter. After covalent coupling the negatively charged moiety with the compound at least one free acid group is required to fulfil the charge requirements as necessary. On the one hand, cleavage of the new chemical bond is necessary to release the original molecule close to or at the target side to display its desired therapeutic effect, for example. On the other side, it is important that the modification should be chemically stable during formulating and *in-vivo* application. Also, chemical stability should be given to efficiently deliver the compound to its target site. The chemically modified therapeutic compound is usually named as prodrug.

Cleavage of the chemical bond between the molecule and the negatively charged moiety, either by enzymes or by simple hydrolysis, should take place when the loaded liposomes reach the target side. On binding and/or endocytosis the chemically modified molecule enters the target cell followed by cleavage of the negatively charged moiety releasing the therapeutically/diagnostically active compound.

Chemical derivatization preferably comprises coupling of a derivatization moiety to a reactive functional group on the low molecular weight compound. Preferred examples of reactive functional groups are, for example, hydroxy groups and amino groups .

Preferred examples of derivatization moieties are anhydrides or halides of polyhydric organic acids, particularly anhydrides of dicarboxylic acids, e.g. succinyl anhydride. The covalent bond between derivatization moiety and compound is preferably a bond which can be cleaved at the desired target site, e.g. by hydrolysis. Preferred bonds are ester or amide bonds.

Alternatively, the derivatization may comprise an intramolecular reaction, e.g. a ring opening reaction, wherein a free negatively charged group is generated. For example, lactone rings may be opened.

Another object of the present invention is to provide a cationic liposome obtainable by the inventive method and further a pharmaceutical composition comprising a pharmaceutically effective amount of said liposome together with a pharmaceutically acceptable carrier, diluent and/or adjuvant.

The inventive liposome can comprise a stabilizing agent such as a cryoprotectant which is selected from a sugar or an alcohol or a combination thereof such as trehalose, maltose, sucrose, glucose, lactose, dextran, mannitol or sorbitol. The cryoprotectant in a liquid formulation can be present in an amount from 1 % (w/v) to 20% (w/v) preferably from 5 % (w/v) to 10% (w/v). Other protective agents are tocopherol, vitamin C, and other substances which protect the formulation from chemical degradation, as an example due to unfavourable light, temperature or other environmental conditions. As an example, the lipophilic tocopherol can be present from about 0.05 mol% to about 0.5 mol %, the water-soluble vitamin C can be present from about 0.05 mM to about 1.0 mM.

The inventive pharmaceutical composition can be used for diagnosing, preventing and/or treating a condition characterized by enhanced angiogenic activity, preferably cancer such as solid tumors and their metastases like bladder, brain, breast, cervical, colorectal, endometrial, head and neck or kidney cancer, leukaemia, liver or lung cancer, lymphoma, melanoma, non-small-cell lung, ovarian, pancreatic or prostate cancer. Further conditions may be a chronic inflammatory disease, rheumatoid arthritis, dermatitis, psoriasis or wound healing.

Further provided is a method of delivery of a low molecular weight compound to an angiogenic vascular target site comprising the steps of
a. providing a pharmaceutical composition comprising a cationic liposome with a low molecular weight compound as an active ingredient,
b. administering the pharmaceutical composition to a subject in need thereof,
c. allowing the pharmaceutical composition to associate with the target site and
d. releasing and optionally activating the low molecular compound.

If the low molecular weight compound is an imaging agent, the composition may either enter the cell once it reaches its site or it may accumulate within the periphery without being taken up by the targeted cells. Furthermore, the imaging agent may leave the liposomal construct upon targeting in order to be used for imaging purposes or it may have diagnostic properties within the liposomal construct. Examples of agents where the disruption of the liposomal membrane is desired upon targeting are MRI contrast agents, whose effectiveness may be reduced by the liposomal membrane's slow water exchange. Here, a release of the contrast agent is preferred. This release may occur upon interaction with the target site or upon endocytosis. Similarly, fluorescent dyes, which are quenched by constituents of the liposomal membrane, induce a greater fluorescence signal upon destruction of the lipid vesicle. On the other hand, radioactive imaging agents will result in an image independently of the fate of their liposomal vehicle, as the radioactive decay is not influenced by the liposomal constituents. For diagnostic purposes in general, uptake of the imaging agent by the targeted cell is not required.

The chemically modified therapeutic compound is usually named as prodrug. Release of the drug from the prodrug may be effected by dissociation of the modification moeity, e.g. by cleavage of the chemical bond between the molecule and the negatively charged moiety, e.g. either by enzymes or by simple hydrolysis. Preferably, the release, should take place when the loaded liposomes are at the target site. On binding and/or endocytosis the chemically modified molecule enters the target cell followed by cleavage of the negatively charged moiety releasing the therapeutically/diagnostically active compound.

Releasing and optionally activating the low molecular weight compound comprises
a) liberating said compound from the associated cationic amphiphile and/or
b) eliminating said negatively charged moiety, e. g. by hydrolysing a covalent bond or by perfoming a ring-closure reaction.

The latter may take place by endocytosis with the target cell and/or by fusion with the target cell membrane and/or by diffusion through the target cell membrane. Endocytosis therein comprises releasing said compound inside the cell.

Activating the prodrug may comprise hydrolysing, ring closuring or any other way of reverting the modification of the compound. Activating means any transformation of the modified compound into the therapeutically/diagnostically active original structure/form.

Further, the invention is explained in more detail by the following Figures and Examples.

### Figures

**Fig. 1****:** UV-vis spectra of fluorescein derivates (AF, F, and CF) before and after association to the liposomes. In the graph, DOTAP indicates DOTAP/DOPC 50/50 and PG indicated DOPG/DOPC 5/95. The free CF curve is identical to the one of AF and F (the latter two spectra are not shown for clarity). As can be seen, with DOPG/DOPC liposomes, no spectral shift is observed. On the contrary, for the DOTAP/DOPC liposomes a spectral shift is obvious, which increases as AF<F<CF.
**Fig. 2****:** UV-vis spectra of a 5 mM solution of AF, mixed 1:1 with a suspension of 25 mM DOTAP/DOPC liposomes. A) after mixing. b) after diafiltration in order to remove the free dye. c) after addition of triton in order to destroy the liposomes and to release the dye from the lipid.
**Figs. 3 and 4****:** Chemical Stability of Succinyl Paclitaxel (SuccTXL) shown as Paclitaxel (TXL) release at different pH and temperature conditions.
**Figs. 5 and 6****:** Physical and Chemical Stability of Liposomal SuccTXL shown as Lipsomal SuccTXL content and TXL release.
**Figs. 7 and 8****:** Stability of Liposomal SuccTXL shown as TXL Release at 4°C and at Room Temperature (RT)
**Fig. 9****:** Treatment of A-375 Melanoma in NMRI Nude Mice with Taxol® and SuccTXL - Cationic Liposomes
**Fig. 10****:** Treatment of Ea.hy926 cells with different Camptothecin Formulations
**Fig. 11:** Treatment of A-375 Melanoma in NMRI Mice with different Camptothecin Formulations
**Fig. 12****:** Excitation and fluorescence spectra of Camptothecin dissolved in chloroform/methanol and in the liposomal form.
**Fig.13**:UV-vis spectra of CPT under different conditions.

### Examples

The following examples shall demonstrate the invention but are not limiting to it.

### A Association Of Water-Soluble Molecules To Liposomes

### 1. Fluorescein Dye Derivatives, Reference Example

As examples, formulation studies with different fluorescein dye derivates are outlined. Formulations with aminofluorescein (AF) fluorescein (F) and carboxy-fluorescein (CF) are presented. All three components have a carboxy-group where the acidity of the proton increases as AF<F<CF, and therefore as well the binding to cationic lipids increases in that sense.

The formulation procedure may consist of the following steps.

### 1.1. Direct Loading

### Formation of the liposomal suspension

### a. Film method

Liposomes may be formed by the well-known film method, such as described in the literature.[1] Briefly, from the organic solution of the lipid, or the lipid plus the drug, the solvent is evaporated, and a thin film of lipid (or lipid plus drug) is formed at the inner wall of a flask. The thin molecular film is resuspended in an aqueous phase, which contains the dye, and which may contain further components such as buffers, ions, cryoprotectants, etc. A typical formulation consists of a suspension of 25 mM DOTAP/DOPC 1:1, which is resuspended by a 5 mM solution of the dye.

### Typical formulations

**Table 1: Formulations with anionic and cationic liposomes.**

| Composition | Total molarity [mM] | lipidMarker | Conc. bef. diafiltration [mg/ml] | markerConc after diafiltration [mg/ml] | marker max (nm) |
|---|---|---|---|---|---|
| DOPG/DOPC 95/5 | 25 | CF | 1.9 | 0.12 | 492.6 |
| DOTAP/DOPC 50/50 | 25 | AF | 1.7 | 0.8 | 497.6 |
| DOTAP/DOPC 50/50 | 25 | F | 1.7 | 1 | 502.2 |
| DOTAP/DOPC 50/50 | 25 | CF | 0.19 | 0.19 | 506 |

The aqueous solution may contain glucose, trehalose and buffers (Tris), to achieve a desired pH after reconstitution. A typical pH is between 7 and 8. However, depending on the lipid, as well higher (up to pH 9) or lower (down to pH 6) values may be selected. The pH of the liposomal formulation may be altered after formulation for further treatment.

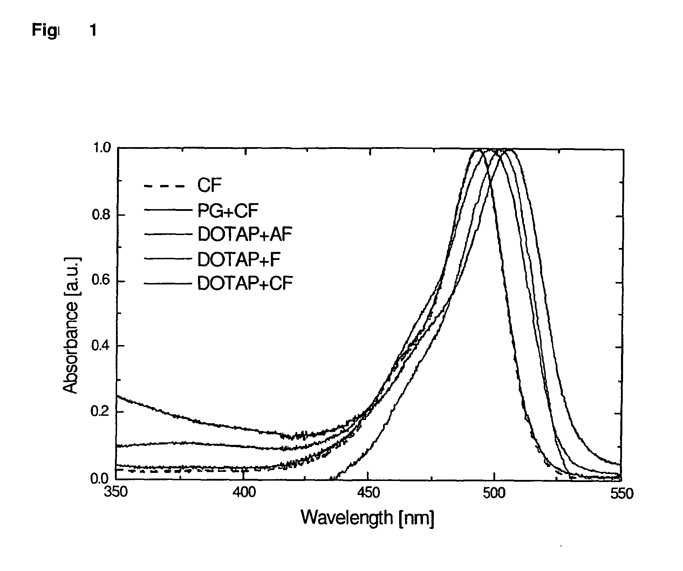

As an example, in table 1 the results from some experiments are given. For comparison as well a formulation with anionic lipids was performed. For the anionic liposomes, most of the dye is lost during diafiltration, while for the cationic liposomes a large molar fraction of the dye remains immobilized in the lioposme suspension. This shows, that the dye molecules bind efficiently to the lipid liposomes. For the CF experiments a lower bulk concentration of the dye was selected,because in that case binding is so strong, that for higher concentrations the liposomes will be destabilized due to the sterical hindrance by the CF molecules. The fraction of dye which was found immobilized in the suspensions from cationic liposomes was wuch higher that for the (anionic) DOPG containing liposomes. A band shift (λ max) in the absorption spectrum was observed, which increased as AF<F<CF, indication increasing binding strength. On the contrary, for the DOPG liposomes, no shift of the absorption maximum occurred. This can be seen most clearly in Fig. 1, where the complete spectra for the free and the liposomal dyes are depicted.

### b. Organic Solution Injection

Alternatively to the film method liposomal suspensions may be achieved by injection of a solution of the lipid into the aqueous solution of the dye [cit.]. The dye concentration can vary in a wide range according to the desired final dye/lipid ratio. A standard value is 5 mM for experiments, where the final lipid concentration is 15 mM. A typical solvent for the lipid is ethanol ('ethanol injection'). Typically the concentration of the lipid in ethanol is 400 mM, however, concentrations different to that value (200 mM, and lower) can be applied as well. In general, the medium size of the resulting liposomes can be controlled to some extent by the lipid concentration. The suitable volume of the lipid solution in ethanol is injected under vigorous stirring, where the injected volume is given by the desired final lipid concentration. Usually, liposomal formulations with lipid concentration between 10 and 25 mM are prepared, but concentrations higher or lower than that are possible as well. All compositions and concentrations as described in 1.1.a can be achieved by this approach. As an alternative to ethanol, as well other suitable solvents or mixtures may be used. Typically, these are alcohols, ethers, chloroform, hydrocarbons, etc. As well solvents in the supercritical state can be applied, such as hydrocarbons, carbon dioxide, perfluorinated compounds, etc. Subsequently to the described formulation procedure, extrusion (2) dialysis (3), a concentration step (4) or freeze drying may be performed.

### Extrusion

The liposomal suspensions as achieved by the above-described methods do not have necessarily the desired size distribution. Therefore, an extrusion through a membrane of defined pore size may be performed subsequently. In our experiments we have usually performed extrusion through membranes of 200 nm pore size (company, specification). Other typical extrusion membranes were with 100 nm or with 400 nm pore size. Size distributions were determined by quasi-elastic light scattering.

### Dialysis

Separation of low molecular compounds from the liposomal suspensions Diafiltration techniques were used to separate low molecular components from the liposomal suspensions. For the above described 15 mM DOTAP/DOPC 1:1 formulations with 5 mM dye in the aqueous phase, ca. 50% of the dye was retained in the liposome suspension for AF, and 60% was retained for F. With 0.5 mM of CF, virtually 100% of the dye was retained in the solution (the amount of released dye was monitored by UV-vis)spectroscopy.

### Freeze-Drying

Freeze drying of the liposomal formulations was performed using standard protocols. The composition and the physical state of the suspensions are characterized after reconstitution such as described below. The suspension as described above could be frozen and brought back to room temperature without drastically affecting the aggregation state of the liposomes.

### 1.2. Remote Loading

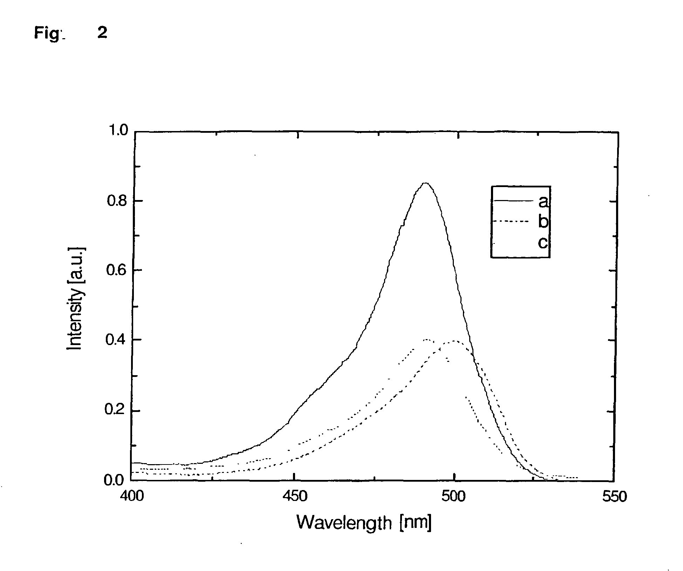

The association of the dye to the liposomes can be performed as well to an already prepared formulation, by simply mixing the dye solution with the liposome suspension. If the dye/lipid ratio does not exceed the maximum loading and if the binding constant is high enough, virtually all of the dye can be linked to the liposomes. The efficacy of the loading can be determined by diafiltration and by UV-vis and fluorescence spectroscopy of the suspension. As an example the results from remote loading of DOTAP/DOPC liposomes with AF (the most weakly binding fluorescein variety) are given. A 25 mM suspension of the pure lipid liposomes was prepared by the standard procedure.Then a 5 mM solution of AF was added to the suspension (equal volumina). Subsequently the suspension was dialyzed in order to remove the free dye. The dye content in the solution was controlled by UV-vis spectroscopy. In fig. 2, the spectra after mixing, after dialysis, and after addition of triton are shown. The triton was added in order to destroy the liposomes and the release the lipid-bound dye.

As can be seen, ca. 50 % of the dye remained bound to the liposomes, and a red shift of the spectrum indicates the lipid-bound state of the dye. After addition of triton, the absorption maximum is shifted back to the original value of the free dye, indicating that the liposomes were destroyed and the dye was released.

### Physico-chemical characterization of the liposomes

All stages of formulation are monitored by HPLC analysis (lipid and drug) and by UV-vis spectroscopy. In HPLC analysis a slight loss of material after extrusion may be observed. UV-vis spectroscopy serves as a qualitative means in order to control formulation efficacy. The UV-vis spectra of the dye are shifted on binding to the lipid. The band shift is a function of the binding constant and the fraction of lipid bound and free dye can be estimated by UV-vis spectroscopy.

Quasi-elastic light scattering (Zetasizer 1000 and Zetasizer 3000, Malvern Instruments) was measured in order to determine the size distribution of the liposomes.
The zeta potential was determined with a Zetasizer 3000 (Malvern Instruments).

### 2. Associating Of Patent Blue with Cationic Liposomes, Reference Example

This example will illustrate that the negatively charged dye patent blue closely associates with cationic liposomes.

### 2.1. Preparation of Liposomes

Patent blue (alphazurine A) is a water-soluble dye approved for diagnostic visualization of the lymph system.

A 2.5% and a 0.2% (w/w) aqueous solution of the dye is prepared. Liposomes are prepared by dissolving the appropriate amount of lipids in chloroform and evaporating the solvent under vacuum until a thin lipid film is formed. The lipid film is dried at 40°C under a vacuum of 3 to 5 mbar for approximately 60 minutes. Subsequently, the lipids are dispersed in the appropriate volume of the patent blue (PB) solution yielding a suspension of multilamellar lipid vesicles (10 mM lipid concentration). One day later, the vesicles are extruded (filtration under pressure) through a 400 nm membrane (5 passes). Table 2 summarizes the formulations prepared.

**Table 2: Liposomal Formulations used in the Example**

| **Formulation** | **lipid composition (mol%)** | **Charge of Liposomes** | **Lipid concentration** | **Lipid Film rehydrated with** |
|---|---|---|---|---|
| A | DOTAP/DOPC 50/50 | positive | 10 mM | 0.2% PB, aqueous solution |
| B | DOTAP/DOPC 50/50 | positive | 10 mM | glucose (aqueous solution) |
| C | EPC/Chol = 80/20 | neutral | 10 mM | 2.5% in aqueous solution |
| D | EPC/Chol = 80/20 | neutral | 10 mM | 0.2% in aqueous solution |

### 2.2. Separation of non-associated dye from neutral liposomes

Gel filtration is employed to separate the dye (MW < 1000 Da) from the colloidal liposome. The free dye is retained in the pores of the column, the liposomes are so large that they elute without retention in the void volume. It is expected that in a liposomal formulation with a lipid concentration of 10 mM approximately 2% of the aqueous phase is entrapped in the liposomes, 98% are not entrapped (Liposomes a practical approach, Ed. R. R. C. New, IRL Press, Oxford 1990 ). This amount of dye is to be separated from the liposome by gel filtration.

A Microspin^{™} S-300 HR column (Amersham) was pre-equilibrated as required in the product description and loaded with 50 µl of the neutral liposomal formulation C. The goal was to investigate whether patent blue not associated with the liposomes can be separated from the liposomal fraction as described in the literature (Reynolds, J. A.; Nozaki, Y.; Tanford, C. Gel-exclusion chromatography on S1000 Sephacryl: application to phospholipid vesicles. Anal Biochem 1983, 130 (2), 471-474.) The majority of the dye was retained on the column, a slightly blue solution eluted (liposomes with associated dye; about 2% as estimated by UV spectroscopy). This illustrates that for neutral liposomes we can apply well-established separation protocols to separate un-associated material.

### 2.3. Separation of non-associated dye from cationic liposomes

Microspin^{™} S-300 HR columns were used to separate the non-associated dye from the liposome formulations A and B. Three columns were pre-equilibrated as required in the product description and loaded with 50 µl of 0.2% solution of PB (column 1), the liposomal formulation A (column 2) or liposomal formulation B to which 0.2% PB solution was added (column 3). The free dye PB was trapped in the pores of the column material as expected. However, for column 2 PB could not be separated from the cationic liposomes and the majority of the material eluted together with the cationic liposomes in the void volume from the column. In the case of the mixture between cationic liposomes and PB (column 3) surprisingly we found that the interaction between the cationic liposomes and the dye is so strong that the simple mixing of the two components dye and liposomes led to interactions so strong that the dye was carried with the liposomes through the column.

### 2.4. Dialysis of liposome-associated PB

The findings from part 2.2 and 2.3 were further confirmed by dialysis. The liposomal formulation A, C and D were dialyzed for 8 hours in a dialysis tube with a molecular weight cutoff (MWCO) of 8-10000 against glucose. The goal of the experiment was to separate un-associated dye from the liposomally associated dye by letting the free PB pass through the membrane pores due to its small molecular weight.

For formulation A, no measurable dye was dialyzed out of the tubing (less than 1% as determined by UV spectroscopy). For formulation C and D, a significant amount of dye was dialyzed (about 60-70%). More dye was removed here after the dialysate was changed. This example underlines again the strong molecular interaction between cationic liposomes and an anionic dye.

### 3. Metal And Halide-Containing Derivates

The following examples will illustrate that negatively charged compounds such as gadolinium complexes, technetium complexes, and fluoride anions closely associate with cationic liposomes. Other anions such as halide ions, boron clusters, or metal complexes of other metal ions such as the paramagnetic and luminescent lanthanides, radioactive tracers such as indium, thallium or gallium may be associated with cationic lipids in a similar way.

### 3.1 Gadolinium-Containing Contrast Agents and Cationic Lipids

The lipids (10 mM DOTAP/DOPC 50/50) are dissolved in chloroform and the lipid solutions transferred into a round bottom flask. The organic solvents are evaporated under vacuum forming a clear lipid film on the flask's surface. This film is dried at 40 °C under vacuum (3 to 5 mbar) for about 60 minutes. Then, the film is rehydrated with the appropriate volume of an aqueous solution of the contrast agents resulting in a liposomal solution. The next day, the liposomal solution is extruded 5 times through a 400 nm membrane. To remove non-liposomal contrast agent the liposomes are dialyzed 3 times for 8 hours against glucose, pH 7 (Pierce, Slide-A-Lyzer Dialysis Cassette; 10000 molecular weight cut off). The size of the liposomes was determined at a 90° angle (25 °C) by photon correlation spectroscopy (PCS) using Zetasizer 1000 and 3000 (Malvern Instruments). The mean diameter obtained by extrusion through 400 nm membranes is 250 nm and the Gd concentration is determined by X-ray fluorescence.

**Table 3: Gd-Containing Liposomes**

| Formulation | Lipid Composition % | Lipid Concentration | Rehydrated with |
|---|---|---|---|
| A | DOTAP/DOPC 50/50 | 10 mM | 300 mOsm/Kg Multihance |
| B | DOTAP/DOPC 50/50 | 10 mM | 300 mOsm/Kg Magnevist |

### 3.2. A Liposome Composed of Fluoride and Cationic Lipids , Reference Example

The lipids (either 10 mM DOTAP or 10 mM DOPC) are dissolved in chloroform and the solution is transferred into a round bottom flask. The organic solvent is evaporated under reduced pressure forming a clear lipid film on the flask's surface. This film is dried at 40 °C under vacuum (3 to 5 mbar) for about 60 minutes. Then, the film is rehydrated with the appropriate volume of an aqueous 150 mM sodium fluoride solution resulting in a liposomal solution. The next day, the liposomal solution is extruded 5 times through a 200 nm membrane. The size of the liposomes is determined by PCS (Zetasizer 1000 and 3000, Malvern Instruments). The fluoride that is not associated with the liposomes is removed by 3 successive dialysis steps (8 hours, Pierce, Slide-A-Lyzer dialysis cassette; 10000 molecular weight cut off) and the liposomal fluoride concentration is established by ion chromatography. For cationic 10 mM DOTAP liposomes the fluoride concentration is 1.3 mM, whereas the fluoride ion concentration is about 4 times lower for neutral 10 mM DOPC liposomes (0.3 mM fluoride). The far lower fluoride ion concentration of neutral liposomes is explained by the attractive forces of cationic lipids interacting with the negatively charged halide anion.

### B Association of Water-Insoluble Molecules to Liposomes

### 1. Synthesis of Paclitaxel Derivatives

Succinyl paclitaxel (SuccTXL) was synthesized based on the procedure by Horowitz [US4942184] or[2].

In the following, examples with succinyl paclitaxel serve as specific example for different derivatives. However, accordingly other negatively charged derivatives are synthesized and formulated using the activated form (carboxylic acid chloride or carboxylic acid anhydride) of lactic acid, citric acid, glutaric acid, maleic acid, fumaric acid, malonic acid, adipic acid or glutamic acid. Paclitaxel (Taxol) (33 mg, 0.0386 mmol) was dissolved in 0.5 ml of dry pyridine to which 7.7 mg of succinic anhydride (0.0772 mmol) and 0.5 mg (0.00386 mmol) of 4-dimethyl aminopyridine were added. The resulting solution was stirred for 3 h at room temperature. The product was purified by chromatography on a silica-gel 60 column to give 34.6 mg of succinyl paclitaxel. Rf was 0.42 in chloroform-methanol (90:10). Yield: 84%.

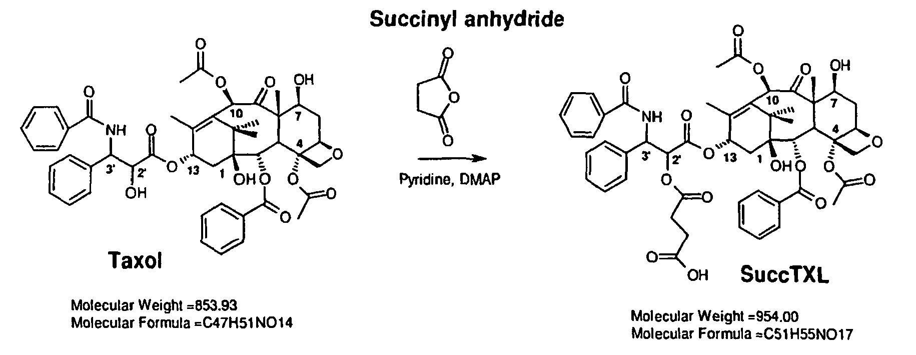

### Structural Characterization:

NMR, TLC and MS analysis confirm the chemical structure of succinyl paclitaxel. The analytical data are consistent with results reported in literature.

The purity is higher than 99% according to HPLC analysis. Some parameters of the applied HPLC method are outlined in the following:

| | |
|---|---|
| Flow rate | 1 mL/min |
| Wavelength | 229 nm |
| Injected Volume | 20 µl |
| Mobile Phase | Acetonitril/THF/2 mM ammonium acetate buffer ( pH 5), 32/12/56 Co(v/v/v), pH 4.7 (adjusted with acetic acid) |
| lumn | LiChroCart 250-4, LiChrospher 60, RP-select B |
| | (Precolumn: 8/4 Lichrospher 100-5 C18) |
| Retention Time | paclitaxel: 17-20 min |
| | succinyl paclitaxel: 7-10 min |

### Synthesis of Succinyl Paclitaxel-DOTAP (SuccTXL-DOTAP)

The SuccTXL-DOTAP is formed by precipitation/crystallization from an organic solution of succinyl paclitaxel and DOTAP as follows:
A methanolic solution of succinyl paclitaxel and DOTAP (N-(2,3-dioleoyloxy-propane)-N,N,N-trimethyl ammonium chloride) at equimolar concentration ratios added to water. After evaporation of the solvent the remaining solid is dissolved in water and freeze-dried to give a white fine powder of the SuccTXL-DOTAP.

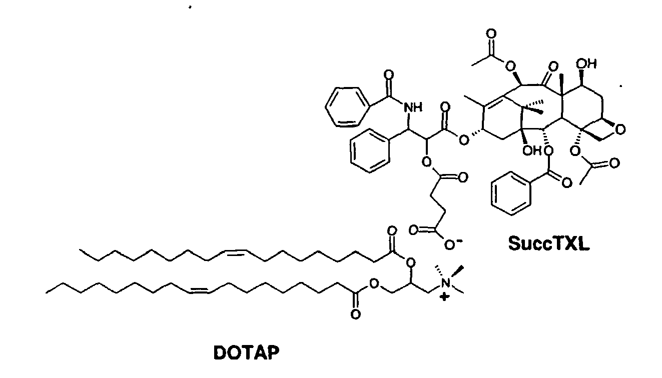

### Chemical analysis:

¹NMR-spectroscopy (Bruker 400 MHz, CDCl3:CD3OD 3:1:TMS); DOTAP related signals: (ppm) δ 0.89 (t, *J*=7.0 Hz, 6H; CH₃), 1.23 (m, 40H; 4-7- und 12-17-CH₂), 1.65 (m, 4H; 3-CH₂), 2.02 (m, 8H; 8- und 11-CH₂), 2.36 (m, 4H; 2-CH₂), 3.20 (s, 9H; N⁺(CH₃)₃), 3.62-3.80 (m, 2H; N-CHH, N-CHH), 4.05 (m, 1H; O-CHH), 4.47 (m, 1 H; O-CHH), 5.34 (m, 4H, 9 and 10-CH), 5.60 (m, 1 H; CH-O); succinyl paclitaxel related signals: (ppm) δ 1.11 (s, 3H, C17-H), 1.19 (s, 3H, C16-H), 1.62 (s, 3H, C19-H), 1.76 (s, 3H, C18-H), 2.2 (m, 2H, C14-H), 2.22 (s, 3H, C10-OAc), 2.43 (s, 3H, C4-OAc), 2.6 (m, 14H, 16H), 2.22 (s, 3H, OAc), 2.43 (s, 3H, OAc), 2.6 (m, 4H, CH CH ), 3.34 (d, 1H, C3-H), 4.17 (d, 1 H, C20-H), 4.48 (d, 1H, C7-H), 4.96 (dd, 1H, C5-H), 5.51 (d, 1H, C29-H), 5.67 (d, 1H, C2-H), 6.21 (t, 1H, C13-H), 6.27 (s, 1H, C10-H), 7.07 (d, 1 H, NH), 7.3-8.1 (m, 15H, arom.). Anal. (C H NO) C, H, N, O

### Synthesis of Further Succinyl Paclitaxel-Cationic Amphiphile (SuccTXL-CA)

Compositions containing succinyl paclitaxel and other cationic amphiphiles are prepared based on the procedure as applied for SuccTXL-DOTAP. SuccTXL-cationic amphiphile composition are prepared with an the cationic amphiphile selected from the following list:
DSTAP N-(2,3-distearyloxy-propane)-N,N,N-trimethyl ammonium chloride
DMTAP N-(2,3-dimyristoyloxy-propane)-N,N,N-trimethyl ammonium chloride
DODAP N-(2,3-dioleoyloxy-propane)-N,N-dimethyl amine
DMDAP N-(2,3-dimyristoyloxy-propane)-N,N-dimethyl amine

¹NMR-spectroscopic and elemental analysis data are consistent with the chemical structures.

### Chemical Stability of Succinyl Paclitaxel

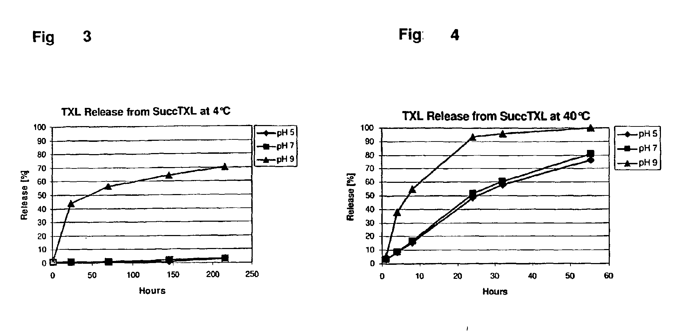

Chemical stability of succinyl paclitaxel in an aqueous solution (at 0.1 mM, Tris-buffered) at different temperatures (4 °C and 40 °C) and at different pH (pH 5, 7, and 9) is determined by HPLC and shown in Figures 3 and 4.

The stability at 4 °C directly correlates with the amount of free paclitaxel that is released from succinyl paclitaxel by cleavage of the formed ester bond between the 2'-OH group of paclitaxel and the negatively charged moiety (succinic acid). At a pH of 5 and 7 succinyl paclitaxel shows high stability with a paclitaxel release of only 4% after 9 day, whereas at pH 9 paclitaxel release is significantly increased to 70% after 9 days.

At a temperature of 40 °C an intensive release of paclitaxel is observed within 56 hours at all pH values indicating that the entire amount of the given chemically bound paclitaxel might be available *in vivo.*

### Liposomal Formulations of SuccTXL-DOTAP

Liposomes are formed by but not limited to lipid film method or ethanol injection.

Liposomal formulations comprising the SuccTXL-DOTAP are prepared using the lipid film method as follows: The lipids and succinyl paclitaxel are dissolved in chloroform in a round bottom flask. The flask is then rotated under vacuum (100 to 200 mbar) until a thin lipids film is formed. The lipid film is thoroughly dried at 40°C under vacuum of about 3 to 5 mbar for approximately 60 minutes. The dry lipid film is cooled in an ice bath and is rehydrated with a cold (4°C) glucose solution resulting in a suspension of multilamellar lipid vesicles at a total concentration of about 10 to 20 mM. Once a homogeneous dispersion is formed (after 15-20 min) the liposomal dispersion is extruded (filtration under pressure) at temperatures between 4° C and 40 °C between 1 and 5 times through polycarbonate membranes of appropriate size, typically between 100 and 400 nm. The formed liposomal dispersion are characterized by measuring the real concentration of each component (HPLC) and determination of the zeta potential and the liposomal size using standardized procedures. Liposomes comprising DOTAP with or without DOPC and SuccTXL-DOTAP are formed with molar ratios as shown in the following tables.

Liposomal formulations containing SuccTXL-DOTAP, DOTAP and DOPC:

| **Formulation** | **Liposomal Composition** | | **Liposomal Size [nm] (PI)** | **Zeta [mV]** | **Potential** |
|---|---|---|---|---|---|
| | Theoretical | Measured | | | |
| DOTAP/DOPC/SuccTXL | 50/47/3 | 50.6/46.1/3.2 | 184 (0.20) | 58 | |
| DOTAP/DOPC/SuccTXL | 50/45/5 | 49.8/44.3/5.9 | 187 (0.16) | 54 | |
| DOTAP/DOPC/SuccTXL | 50/43/7 | 50.8/43.1/6.1 | 170(0.11) | 53 | |
| DOTAP/DOPC/SuccTXL | 50/41/9 | 50.7/40.8/8.5 | 178 (0.19) | 53 | |
| DOTAP/DOPC/SuccTXL | 50/39/11 | 49.7/39.0/11.3 | 175 (0.16) | 50 | |
| DOTAP/DOPC/SuccTXL | 50/37/13 | 51.0/37.7/11.3 | 161 (0.10) | 52 | |
| DOTAP/DOPC/SuccTXL | 50/35/15 | 51.7/35.6/12.7 | 171 (0.22) | 50 | |
| DOTAP/DOPC/SuccTXL | 50/33/17 | 52.5/33.6/13.9 | 205 (0.50) | 50 | |

Liposomal formulations containing SuccTXL-DOTAP and DOTAP:

| **Formulation** | **Liposomal Composition** TheoreticalMeasured | | **Liposomal Size [mn] (PI)** | **Zeta [mV]** | **Potential** |
|---|---|---|---|---|---|
| DOTAP/SuccTXL | 89/11 | 89.7/10.3 | 185 (0.40) | 62 | |
| DOTAP/SuccTXL | 85/15 | 86.9/13.1 | 162 (0.10) | 60 | |

Employing the lipid film method and the DOTAP/DOPC system, liposomes with up to 14mol% (according to SuccTXL) can be formulated as measured by HPLC. The zeta potential values decrease with an increase of the SuccTXL-DOTAP content from 3 up to 17 mol% (according to SuccTXL). However, this trend is not observed for formulations without DOPC. The liposomal size is not significantly affected by the liposomal composition.

Liposomes are also prepared using the ethanol injection method:

A solution of DOTAP, DOPC and SuccTXL-DOTAP in ethanol is prepared with different total concentrations such as of 100 mM, 200 mM or 400 mM. Each formulation is characterized by liposomal size measurements shown in the following table:

Liposomal formulations prepared via ethanol injection

| **Composition of the Ethanol Stock Solution** | **Liposomal Size (PI) of the Final Formulation** |
|---|---|
| DOTAP/DOPC/succinyl paclitaxel (50:39:11): | 170 nm (0.7) |
| 70. 2 mg DOTAP | |
| 62.3 mg DOPC | |
| 20.9 mg succinyl paclitaxel | |
| dissolved in 1 ml ethanol (final conc.: 100 mM) | |
| DOTAP/succinyl paclitaxel (89:11): | 180 nm (0.6) |
| 139,1 mg DOTAP | |
| 42,6 mg succinyl paclitaxel | |
| dissolved in 1 ml ethanol (final conc.: 200 mM) | |
| DOTAP/DOPC/succinyl paclitaxel (50:39:11): | 170 nm (0.2) |
| 280.8 mg DOTAP | |
| 249.2 mg DOPC | |
| 83.6 mg succinyl paclitaxel | |
| dissolved in 1 ml ethanol (final conc.: 400 mM) | |

### Physical and Chemical Stability of the Liposomal SuccTXL-DOTAP Formulations

In order to investigate the stability SuccTXL-DOTAP containing liposomes are stored up to 30 days at 4°C. At various time point the concentration of each component is determined (HPLC) and the formulations are characterized by liposomal size, zeta potential measurements and by determination of possible precipitation (microscopic determination). In addition, the chemical stability of succinyl paclitaxel is studied by determination of the released paclitaxel based on HPLC analysis.

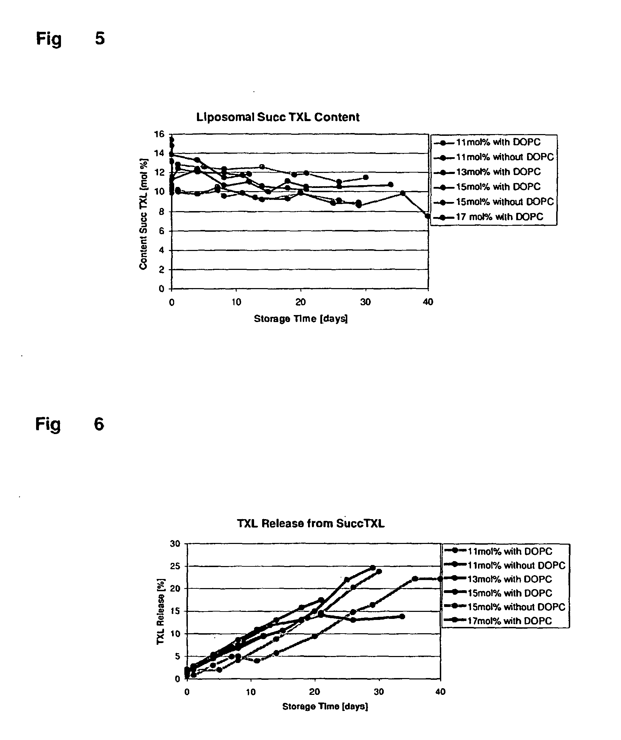

Long-time stability of various liposomal formulations monitored by the SuccTXL content (mol%) and the paclitaxel released from SuccTXL is shown in Figures 5 and 6.

The liposomal SuccTXL-DOTAP content refers to the total amount of lipid and is given as mol% of SuccTXL content. The paclitaxel release from the SuccTXL component of SuccTXL-DOTAP is calculated based on the initial amount of the SuccTXL component in the liposomes and is given as mol%. The results indicate very stable liposomal SuccTXL-DOTAP formulations with and without DOPC. There is a linear time-dependent increase of free paclitaxel with 30mol% release after 30-40 days storage. Precipitation is found whenever the released paclitaxel content reaches a concentration of 0.3 mM (10mM of total lipid). This is consistent with observations with paclitaxel liposomes where an increase of the liposomal paclitaxel amount above a drug to lipid ratio of 3 mol% (0.3mM, 10mM total lipid) results in precipitation.

### Lyophilization of SuccTXL-DOTAP Containing Liposomes

For lyophilization of formulations containing SuccTXL-DOTAP liposomes are prepared by the lipid film method or by ethanol injection (as described above) or another suitable method. Trehalose or another sugar or alcohol is used as cryoprotectant for formulating. A typical lyophilization protocol is shown in the following scheme:

Some examples of liposomal formulations that are lyophilized followed by reconstitution with the necessary volume of water are listed in the following table:

| **Formulation** | **Liposomal Composition** | | **Liposomal Size [nm] (PI)** | **Zeta [mV]** | **Potential** |
|---|---|---|---|---|---|
| | Theoretical Measured | | | | |
| DOTAP/DOPC/SuccTXL (10 mM total conc.) | 50/39/11 | 49.7/39.0/11.3 | 196(0.03) | 55 | |
| DOTAP/DOPC/SuccTXL (20 mM total conc.) | 50/39/11 | 49.7/40.5/9.8 | 200(0.13) | 54 | |
| DOTAP/SuccTXL (10 mM total conc.) | 89/11 | 90.5/9.5 | 179(0.09) | 58 | |
| DOTAP/SuccTXL (20 mM total conc.) | 89/11 | 88.2/11.8 | 209(0.16) | 64 | |

Both, liposomal formulation with and without DOPC are easily formed by reconstitution of the lyophilisates as shown by PCS and zeta potential measurements. In addition, no significant paclitaxel release is observed during the lyophilization procedure as indicated by the HPLC data.

### Stability of Lyophilized SuccTXL-DOTAP Containing Liposomes

The stability of freshly reconstituted lyophilized liposomes is studied at 4 °C and at room temperature over a period of 24 hours. The composition of the tested formulations is shown in the following table:

| **Formulation** | **Liposomal Composition** |
|---|---|
| DOTAP/DOPC/SuccTXL (10 mM total conc.) | 50/39/11 |
| DOTAP/DOPC/SuccTXL (20 mM total conc.) | 50/39/11 |
| DOTAP/SuccTXL (10 mM total conc.) | 89/11 |
| DOTAP/SuccTXL (20 mM total conc.) | 89/11 |

After adding the adequate amount of water to each lyophilisate, gently shaking followed by storing the reconstituted samples at 4°C for additional 30 min to allow total degasification. The reconstituted liposomes are kept at 4°C or room temperature, respectively. At different time points (0h, 4h, 8h and 24h) the liposomes are characterize by size, zeta potential and determination of the concentration of each component by HPLC.

After 24h storage at 4°C or room temperature each liposomal formulation has liposomal diameters that are nearly identical to the liposomal size prior to lyophilization. In addition, no significant change of the zeta potential of each formulation are observed after the same treatment.

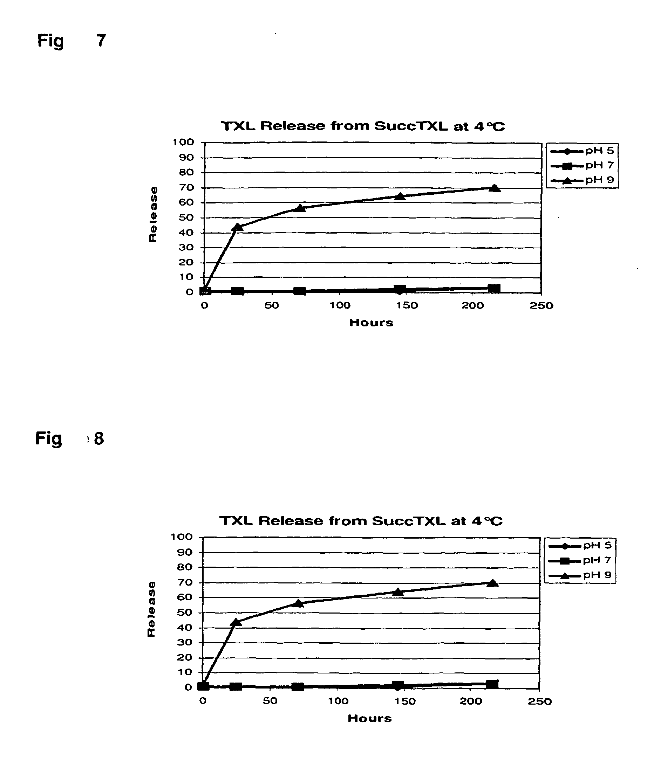

HPLC analysis confirms excellent stability. While the concentration of the lipid components does not change at all, none or only very little paclitaxel release is found after 24 h storage. When stored at 4°C none of the formulations reveals a significant instability of liposomal ccTXL-DOTAP as monitored by measuring the amount of paclitaxel released from SuccTXL (SuccTXL-DOTAP) as shown in Figure 7.

Storage at room temperature results in a formation of paclitaxel of 10-13 mol% according the initial concentration of the SuccTXL as shown in Figure 8.

### In Vitro Experiments

The efficacy of the liposomal succinyl paclitaxel formulation is determined in vitro by analysing the decrease of cell viability in correlation to the drug concentration. The drug concentration at which cell viability is inhibited to 50 % (IC₅₀) is used as index for the inhibitory potential.

A-375 (transformed human endothelial cell line) and Ea.Hy cells (human dermal melanoma cell line) is seeded at a constant density (2x 10⁴/cm²) in 24-well plates and cultivated over night at conditions of 5-5.5 % CO₂, 37 °C and ~90% humidity. At day 1, cell culture medium is replaced by a mixture of fresh medium and a series of 11 consecutive drug dilutions is added to each well (duplicates) to cover a range between 0.1 and 1000 nM final drug concentration. After 72 h, the cell viability in each well is determined by measuring the activity of mitochondrial dehydrogenases (MTT assay). In viable cells the MTT substrate is converted to a blue, cell impermeable dye (Formazan). After 1 h the medium is removed, cells are lysed with isopropanol/0.04 % HCl and the amount of the blue Formazan given as optical density at a wavelength of 550 nm (OD₅₅₀ₙₘ) quantitated in an ELISA reader. The experiment is evaluated using the Sigma Plot analysis software by plotting the mean OD₅₅₀ₙₘ value against the respective drug concentration. A best fit curve is calculated based on a double-sigmoid assumption algorithm and the IC₅₀ value is determined according to this best fit curve with the following result:

| **Formulation** | **IC₅₀ (A-375)** | **IC₅₀ (Ea.Hy)** |
|---|---|---|
| Taxol^{®} | 2 nM | 3 nM |
| succinyl paclitaxel | 10 nM | 17 nM |
| DOTAP/DOPC/succinyl paclitaxel (50/39/11) | 12 nM | 28 nM |

### In Vivo Experiments

NMRI-nude mice from Elevage Janvier and housed in isolated ventilated cages under save environmental conditions (SPF facility, 22°C, 30 - 70% humidity, 12 h light/dark cycle) with food and water *ad libitum.* Experimental design was reviewed and approved by local government.

Tumor cells (A-375 human melanoma cell line, ATCC Nr.: CRL-1619) were grown as described in the data sheet supplied by ATCC. Tumor cells (5 x 10⁶ in PBS) were inoculated s.c. in the right dorsal flank of mice in a volume of 50 µl on day 0.

Mice were assigned to the experimental groups (8 animals per cage), housed and handled (including monitoring of the body weight gain) at least five days before tumor inoculation (= day -6 to 0). Drug treatment begins after the tumors reached a volume of approximately 100 mm³. The drugs were given by iv injection, three times a week (Mo, Wed, Fri) for the following three weeks at equitoxic doses. The drugs were prepared as described. Equitoxic doses of the compounds were determined in separate experiments, where toxicity was evaluated based on haematological parameters, body weight and animal clinical observations. The solutions were administered slowly in a volume of ~10 µl/g body weight.

Animals were clinically monitored during the whole experiment and for at least one week after treatment was finished. Monitoring of tumor size was performed three times a week after staging, before application during treatment period and during recovery period (at least one week). The tumor dimensions were measured by calliper and the tumor size was calculated according to the following formula: V = π L W² / 6 (L = greatest length, W = width of perpendicular axis). The body weight of individual animals was monitored at least twice during handling period (e.g. day -6 and 0), after tumor inoculation, after start of treatment and during recovery period (at least one week) for all groups. EDTA blood was collected from the retrobulbar plexus at four different points: during handling (day -3), tumor staging (day 7), in the middle of treatment (-day 21) and at the end of the recovery period (day 28) from 4 animals of all treatment groups for hematology. The number of red and white blood cells and platelets were determined using an automated cell counter (Abbott Cell Dyn 3500).

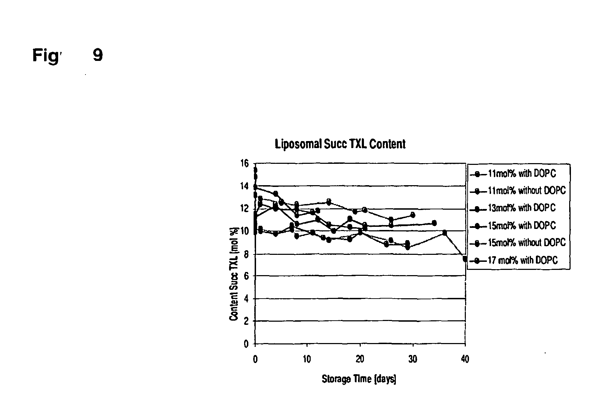

Results of the animal experiments are shown in the scheme and table below. Whereas tumors in the control group showed a rapid and progressive tumor growth, all drug formulations reduced the tumor growth rate. Whereas both SuccTXL-DOTAP formulations showed a strong reduction in the tumor growth rate, Taxol^{®} reduced the tumor growth only to a limited extent (Fig. 9).

| **Group** | **Drug/Formulation** | **Dose [mg/kg]** | **Animals per Group** |
|---|---|---|---|
| 0 | 5 % Glucose | - | 8 |
| 1 | LipoSpa 50 DOTAP/DOPC/succinyl paclitaxel (50/39/11) | 12.5 | 8 |
| 2 | LipoSpa 89 DOTAP/DOPC/succinyl paclitaxel (89/11) | 10.0 | 8 |
| 3 | Taxol^{®} | 5.0 | 8 |

### 2. Synthesis of Camptothecin Derivatives

Succinyl camptothecin (SuccCam) is synthesized based on the procedure used for the synthesis of succinyl paclitaxel. Camptothecin (13 mg, 0.0386 mmol) is dissolved in 0.5 ml of dry pyridine to which 7.7 mg of succinic anhydride (0.0772 mmol) and 0.5 mg (0.00386 mmol) of 4-dimethyl amino pyridine are added. The resulting solution was stirred for 3 h at room temperature. The product is purified by chromatography on a silica-gel 60 column.

### Structural Characterization:

NMR, TLC and MS analysis are used to confirm the chemical structure of succinyl camptothecin.

### Synthesis of Succinyl Camptothecin-DOTAP (SuccCam-DOTAP)

The SuccCam-DOTAP is formed by precipitation/crystallization from an organic solution of SuccCam and DOTAP as follows:

A methanolic solution of SuccCam and DOTAP (N-(2,3-dioleoyloxy-propane)-N,N,N-trimethyl ammonium chloride) at equimolar concentration ratios added to water. After evaporation of the solvent the remaining solid is dissolved in water and freeze-dried to give a white fine powder of the SuccCam-DOTAP. Chemical analysis (NMR, elemental analysis) is used to confirm the SuccCam-DOTAP molar ratio.

### Synthesis of Further Succinyl Camptothecin-Cationic Amphiphile (SuccCam-CA)

Compositions containing SuccCam and other cationic amphiphiles are prepared based on the procedure as applied for SuccCam-DOTAP. SuccCam-cationic amphiphile composition are prepared with an the cationic amphiphile selected from the following list:

| | |
|---|---|
| DSTAP | N-(2,3-distearyloxy-propane)-N,N,N-trimethyl ammonium chloride |
| DMTAP | N-(2,3-dimyristoyloxy-propane)-N,N,N-trimethyl ammonium chloride |
| DODAP | N-(2,3-dioleoyloxy-propane)-N,N-dimethyl amine |
| DMDAP | N-(2,3-dimyristoyloxy-propane)-N,N-dimethyl amine |

### Liposomal Formulations of SuccCam-DOTAP

Liposomes are formed by but not limited to lipid film method or ethanol injection.

Liposomal formulations comprising the SuccCam-DOTAP are prepared using the lipid film method as follows: The lipids and SuccCam are dissolved in chloroform in a round bottom flask. The flask is then rotated under vacuum (100 to 200 mbar) until a thin lipids film is formed. The lipid film is thoroughly dried at 40 °C under vacuum of about 3 to 5 mbar for approximately 60 minutes. The dry lipid film is cooled in an ice bath and is rehydrated with a cold (4°C) glucose solution resulting in a suspension of multilamellar lipid vesicles at a total concentration of about 10 to 20 mM. Once a homogeneous dispersion is formed (after 15-20 min) the liposomal dispersion is extruded (filtration under pressure) at temperatures between 4 °C and 40 °C between 1 and 5 times through polycarbonate membranes of appropriate size, typically between 100 and 400 nm. The formed liposomal dispersion is characterized by determination of the zeta potential and the liposomal size using standardized procedures. Liposomes comprising DOTAP and SuccCam or DOTAP/DOPC and SucCam are formulated in the same manner. In the latter case, a standard ratio DOTAP/DOPC is 50/50 or 50/(50-x) with x the amount of SuccCam in mol%.

Employing the lipid film method and the DOTAP/DOPC system, liposomes with up to 15mol% (according to SuccCam) can be formulated as measured by HPLC. Liposomes with 20 mol% may have unfavourable liposomal size and size distribution (PI). The zeta potential values decrease with an increase of the SuccCam-DOTAP content from 3 up to 20 mol% (according to SuccCam). The liposomal size is not significantly affected by the liposomal composition.

Liposomes are also prepared using the ethanol injection method:. A solution of DOTAP, DOPC and SuccCam-DOTAP in ethanol is prepared with a total concentration of 400 mM. As an example for a formulation with 10 mol% SuccCam, 280.8 mg DOTAP, 253.2 mg DOPC, 35.6 mg SuccCam are dissolved in 1 ml ethanol (final conc.: 400 mM. The ethanolic solution is injected under vigorous stirring into the aqueous phase to give the desired lipid concentration. Most frequently, lipid concentrations of 10 mM to 25 mM are realized.

### Lyophilization of SuccCam-DOTAP Containing Liposomes

For lyophilization of formulations containing SuccCam-DOTAP liposomes are prepared by the lipid film method or by ethanol injection (as described above) or another suitable method. Trehalose or another sugar or alcohol is used as cryoprotectant.

### 2. Camptothecin-Carboxylate Containing Liposomes

### Formation of the liposomal suspension

### a. Film method

Liposomes comprising camptothecin [3] may be formed by the well-known film method as described in the literature. Briefly, from the organic solution of the lipid, or the lipid plus the drug, the solvent is evaporated, and a thin film of lipid (or lipid plus drug) is formed at the inner wall of a flask. The thin molecular film is resuspended in an aqueous phase, which may contain further components such as buffers, ions, cryoprotectants, etc. With this procedure liposomal suspensions are formed in a self-assembly process. A standard formulation is obtained by forming a film of 90 µM DOTAP and 10 µM camptothecin from a solution in chloroform/methanol, 10/1. The film is then reconstituted with 10 ml of the aqueous phase, in order to achieve a suspension where the total liposomal concentration (lipid + drug) is 10 mM. The aqueous solution contains glucose or trehalose and buffers (Tris), to achieve a desired pH after reconstitution. A typical pH is between 7 and 8. However, depending on the lipid, as well higher (up to pH 9) or lower (down to pH 6) values can be selected. The pH of the liposomal formulation may be altered after formulation for further treatment. Thus, a liposomal formulation with the drug/lipid ratio of 1:9, and with a total (lipid+drug) concentration of 10 mM is obtained. Other typical molarities are 15 mM, 20 mM or 25 mM. If necessary, molarities up to 50 mM may be formulated. The drug:lipid ratio usually is selected to be in the rage from 5:95 to 15:85. The lipid phase may consist of only cationic lipids, such as DOTAP, or it may consist up to 50% of charged and non-charged co-lipids. Standard formulations, which we have used frequently, consist of CPT/DOTAP/DPOC = 5:47.5:47.5, or 5:55:45 or 10:45:45 or 10:60:30.

Alternatively to procedure 1a. camptothecin is transformed into the carboxylate, or the carboxylate salt for formulation. Thus, the CPT is 'activated' for easier association of the drug to the cationic lipid, as, in fact, the carboxylate is the molecular form, which associates to the latter. CPT is solubilized in NH₃ and the volume, which contains the necessary amount of the drug (i.e., 10 µM in the example described in 1 a), is added to a flask. After evaporation of the solvent a film of the ammonium salt of the CPT carboxylate is formed. Then the organic solution of the lipid is added and the solvent is evaporated. Thus a camptothecin and lipid film is achieved, where the camptothecin binds more readily to the cationic lipid. Particularly for low pH values, it is easier to obtain liposomes with high drug/lipid values.

Further, the CPT carboxylate may be part of the aqueous solution for reconstitution of the pure lipid film. In that case, a separation step or a concentration step may be performed after formulation.

### b. Organic Solution Injection

Alternatively, liposomal suspensions may be achieved through the injection of a solution of the lipid, or the lipid and the drug, into the aqueous solution. A typical solvent for the lipid, or the lipid/drug phase is ethanol ('ethanol injection').

Ethanol injection of the lipid into the aqueous solution of the drug.

Ethanol injection is a well-known method of the formation of liposomal suspensions. In the present example, ethanol injection into the aqueous solution of the camptothecin-carboxylate is performed. Due to the attractive interaction between the drug and the cationic lipid, camptothecin-containing liposomes are formed in a self-assembly process. In order to achieve 10 ml of a 10 mM suspension of CPT/DOTAP 1:9, a 10 µM solution of CPT-carboxylate, optionally containing further additives as outlined in a. is prepared. Preferentially, the ammonium salt of the CPT carboxylate, such as described in a. is taken. Alternatively, the CPT carboxylate can be formed by dissolving CPT at high pH in another base, and subsequently the pH is brought to the desired value by buffers. The aqueous phase may have a pH between 5 and 8, according to the necessities of the experiment. The suitable volume of the lipid solution in ethanol is injected under vigorous stirring. All compositions and concentrations as described in a. can be achieved by this approach. As an alternative to ethanol, as well other suitable solvents or mixtures thereof can be taken. Typically, these are alcohols, ethers, chloroform, hydrocarbons, etc. As well solvents in the supercritical state can be applied, such as hydrocarbons, carbon dioxide, perfluorinated compounds, etc. Subsequently to the described formulation procedure, extrusion (2) dialysis (3), a concentration step (4) or freeze-drying may be performed.

Alternatively, both the lipid(s) and the drug may be dissolved in the injection solution. All other steps are equivalent.

### Extrusion

The liposomal suspensions as achieved by the above-described methods do not have necessarily the desired size distribution. Therefore, an extrusion through a membrane of defined pore size may be performed subsequently. In our experiments we have usually performed extrusion through membranes of 200 nm pores size (company, specification). Other typical extrusion membranes were with 100 nm or with 400 nm pore size. Size distributions were controlled by quasi-elastic light scattering.

### Separation of low molecular compounds from the liposomal suspensions

Dialysis was used to separate low molecular components from the liposomal suspensions. This was particularly the case for the experiments with ethanol injection, where a fraction of non-liposomal camptothecin and a certain amount of ethanol were present in the suspension. In that case, the release of the free CPT was monitored by UV-vis spectroscopy. For the above described DOTAP/CPT 90:10 formulations by ethanol injection (2b), 80 % of the CPT was found to be bound to the liposomal phase, i.e. 20% was released on dialysis. Equilibrium was reached after 4 h.

Freeze drying of the liposomal formulations was performed using standard protocols. The composition and the physical state of the suspensions were characterized after reconstitution as described below. The suspension as described above could be frozen and brought back to room temperature, without drastically affecting the aggregation state of the liposomes.

### Physico-chemical characterization of the liposomes

All stages of formulation are monitored by HPLC analysis (lipid +drug) and by UV-vis spectroscopy. In HPLC analysis a slight loss of material after extrusion may be observed. UV-vis spectroscopy serves as a qualitative means in order to control formulation efficacy:

It is well known, that the spectrum of CPT depends strongly on the molecular state and on the local environment. Therefore, qualitatively, the spectra of the lactone form, the carboxylate and the liposomal CPT can be distinguished from the shape of the spectra.

Quasi-elastic light scattering (Zetasizer 1000 and Zetasizer 3000, Malvern Instruments) was measured in order to determine the size distribution of the liposomes.

The zeta potential was determined by a Zetasizer 3000 (Malvern Instruments).

### Results from cell culture studies

### In vitro determination of the cytostatic potential of LipoCam

The efficacy of a cytostatic drug is determined in vitro by analysing the decrease of cell viability in correlation to the drug concentration. The drug concentration at which cell viability is inhibited to 50 % ("IC₅₀") is used as index for the inhibitory potential of a respective drug. The higher the cytostatic potential, the lower is the IC₅₀ value. Drugs with high inhibitory potential have IC₅₀ values in the nM range. Here we compared 3 formulations of liposomally encapsulated camptothecin carboxylate (RM544=LipocamI, RM541=LipocamII, RM542 = Lipocam III) with the free carboxylate (RM543).

### Principle

A suitable cell line (i.e. endothelial or tumor cell line) is seeded at constant densities in four 24-well plates. After one or two days, a series of 11 consecutive drug dilutions is added to cover the range between 0 - 5000 nM final drug concentration. Each individual concentration is measured in 2 wells independently to increase the accuracy of the assay. Two wells without drug serve as control. The cells are incubated at optimal growth conditions (5-5.5 % CO₂, 37°C, ~ 90% humidity). After 72h, the cell viability in each well is determined by measuring the activity of mitochondrial dehydrogenases (MTT assay). In viable cells the MTT substrat is converted to a blue, cell impermeable dye (Formazan). After 1 h, the medium is removed, cells are lysed with isopropanol/0,04% HCl and the amount of the blue Formazan quantitated in an ELISA reader at 550 nm (measured against lysis buffer as blank). The experiment is evaluated using the Sigma Plot analysis software by plotting the mean OD₅₅₀ₙₘ value (of the 2 wells containing the identical drug concentration) against the respective drug concentration. The IC₅₀ concentration is taken at the half-maximal OD₅₅₀ₙₘ value. A high inhibitory potential results in a low IC₅₀ value.

### Experiment

2x 10⁴/cm² Ea.Hy926 cells (transformed human endothelial line) are seeded into four 24-well plates and cultivated over night. At day 1, a series of eleven 5x concentrated master dilutions of each camptothecin formulation in cell culture medium is prepared (25000, 10000, 5000, 2500, 1250, 500, 250, 50, 25, 12.5, 5 nM). Having prepared all dilutions, the culture medium is removed from the cells and 400 µl of fresh culture medium + 100 µl of the respective master drug concentration is added (each concentration to 2 wells). This results in a 1:5 dilution of the master series (final drug concentrations between 1 nm and 5000 nM). To two wells no drug is added (controls). The plates are cultivated further 72 h. After this incubation period, the medium is replaced by fresh medium containing the MTT substrat and incubated for 1 h. The medium is removed, the cells are lysed in isopropanol/0.04% HCl and the OD at 550 nm is measured.

### Result

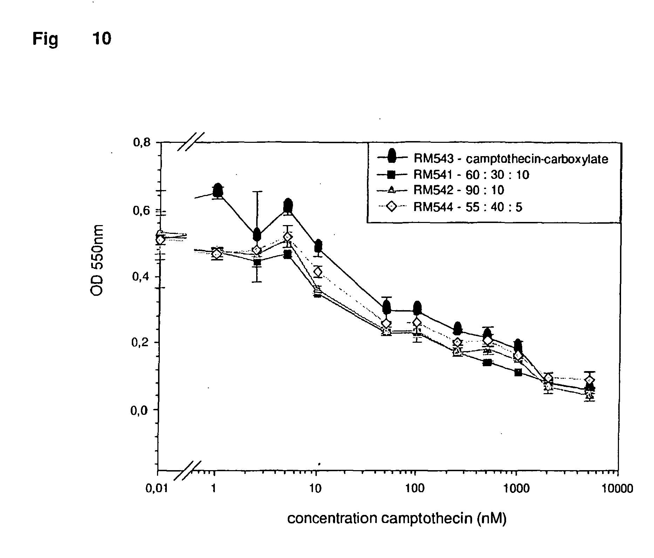

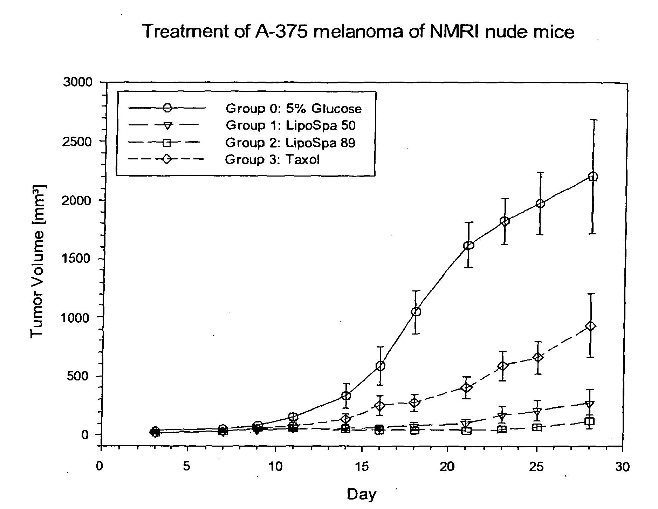

As demonstrated in Figure 10, all three LipoCam formulations (RM541, RM542, RM544) display a comparable or better growth inhibition curve than free camptothecin carboxylate. The IC₅₀ values are around 25-30 nM. Therefore, liposomally encapsulated camptothecin carboxylate has a high cell inhibitory potential in vitro.

### Results from animal studies:

### Therapeutic efficacy of LipoCam in A-375 melanoma of nude mice

### Materials and methods :

NMRI-nude mice were purchased from Elevage Janvier and housed in isolated ventilated cages under save environmental conditions (SPF facility, 22°C, 30 - 70% humidity, 12 h light/dark cycle) with food and water ad *libitum.* Experimental design was reviewed and approved by local government.

Tumor cells (A-375 human melanoma cell line, ATCC Nr.: CRL-1619) were grown as described in the data sheet supplied by ATCC. Tumor cells (5 x 10⁶ in PBS) were inoculated s.c. in the right dorsal flank of mice in a volume of 50 µl on day 0.

Mice were assigned to the experimental groups (8 animals per cage), housed and handled (including monitoring of the body weight gain) at least five days before tumor inoculation (= day -6 to 0). Drug treatment begins after the tumors reached a volume of approximately 100 mm³. The drugs were given by iv injection, three times a week (Mo, Wed, Fri) for the following three weeks at equitoxic doses. The drugs were prepared as described in example. Equitoxic doses of the compounds were determined in separate experiments, where toxicity was evaluated based on haematological parameters, body weight and animal clinical observations. The solutions were administered slowly in a volume of ~10 µl/g body weight.

Animals were clinically monitored during the whole experiment and for at least one week after treatment was finished. Monitoring of tumor size was performed three times a week after staging, before application during treatment period and during recovery period (at least one week). The tumor dimensions were measured by calliper and the tumor size was calculated according to the following formula: V = π L W² / 6 (L = greatest lengt, W = width of perpendicular axis). The body weight of individual animals was monitored at least twice during handling period (e.g. day -6 and 0), after tumor inoculation, after start of treatment and during recovery period (at least one week) for all groups. EDTA blood was collected from the retrobulbar plexus at four different points: during handling (day -3), tumor staging (day 7), in the middle of treatment (-day 21), and at the end of the recovery period (day 28) from 4 animals of all treatment groups for hematology. The number of red and white blood cells and platelets were determined using an automated cell counter (Abbott Cell Dyn 3500).

The results are shown in Figure 11. Whereas tumors in the control group showed a rapid and progressive tumor growth, all drug formulations - applied at equitoxic doses - showed an anti-tumor effect. The most effective formulation was LipoCam III, with total tumor regression in most animals. LipoCam I and LipoCam II showed also a temporary tumor regression with a slow re-growth of tumors beginning in the second half of the treatment period. CPT-Na-Carboxylate showed only a retardation as compared to the control group.

| **Group** | **Drug** | **Dose [mg/kg]** | **N° of mice** |
|---|---|---|---|
| **0** | Glucose | / | **8** |
| **1** | LipoCam I | **5** | **8** |
| **2** | LipoCam II | **5** | **8** |
| **3** | LipoCam III | **2,5** | **8** |
| **4** | CPT-Na-Carboxylate | **1,0** | **8** |

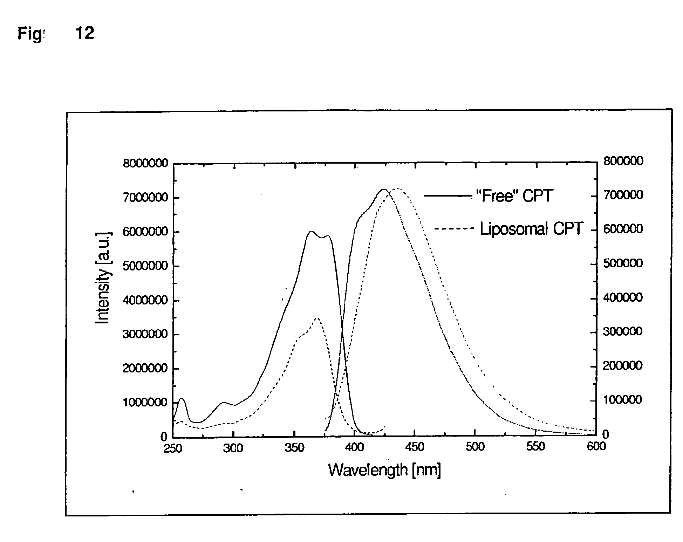

Spectroscopic characterization, excitation and fluorescence spectra of CPT dissolved in chloroform/methanol ("free CPT") and liposomal CPT are shown in Figure 12. Both spectra are different.

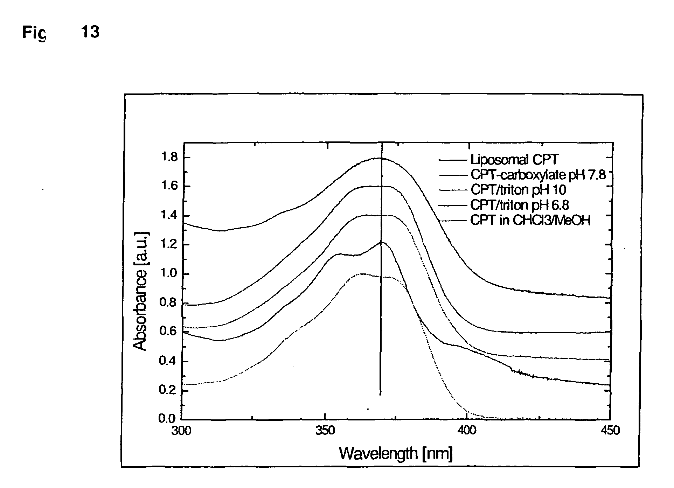

Figure 13 shows UV-vis spectra of CPT under different conditions.

The spectrum of the liposomal CPT (top curve) differs clearly from those under all other conditions. Characteristic shapes can be made out for the liposomal CPT, the CPT-carboxylate (second and third curve from above) and the lactone from of the CPT (fourth and fifth curve from above). The lactone spectrum shows as well a strong dependence on the solvent environment. For clarity the spectra are vertically shifted.

| Typical Formulations | | | | | |
|---|---|---|---|---|---|
| Formulation | Composition DOTAP/DOPC/CPT | Total molarity [mM] | Size [nm] | PI | Zeta potential |
| A | 90/10 | 15 | | 0.157 | 65 |
| B | 55/40/5 | 15 | | | |
| C | 30/30/10 | 15 | 193 | | 51 |

### C Human Therapy Treatment Protocols

This example is concerned with human treatment protocols using the formulations disclosed. Treatment will be of use for diagnosing, preventing and/or treating various human diseases and disorders associated with enhanced angiogenic activity. It is considered to be particularly useful in anti-tumor therapy, for example, in treating patients with solid tumors and hematological malignancies or in therapy against a variety of chronic inflammatory diseases such as psoriasis.

A feature of the invention is that several classes of diseases and/or abnormalities are treated without directly treating the tissue involved in the abnormality e.g., by inhibiting angiogenesis the blood supply to a tumor is cut off and the tumor is killed without directly treating the tumor cells in any manner.

As discussed above, other therapeutic agents could be administered either simultaneously or at distinct times. One may therefore employ either a pre-mixed pharmacological composition or "cocktail" of the therapeutic agents, or alternatively, employ distinct aliquots of the agents from separate containers.

The various elements of conducting a clinical trial, including patient treatment and monitoring, will be known to those of skill in the art in light of the present disclosure.

For regulatory approval purposes, it is contemplated that patients chosen for a study would have failed to respond to at least one course of conventional therapy and would have objectively measurable disease as determined by physical examination, laboratory techniques, or radiographic procedures. Such patients would also have no history of cardiac or renal disease and any chemotherapy should be stopped at least 2 weeks before entry into the study.

Prior to application some formulations have to be diluted with sterile 5% glucose solution in a ratio of 1:3,33. The required application volume is calculated from the patient's body weight and the dose schedule.

Prior to application, the formulation can be reconstituted in an aqueous solution in case the formulation was freeze dried. Again, the required application volume is calculated from the patient's body weight and the dose schedule.

The disclosed formulations may be administered over a short infusion time. The infusion given at any dose level should be dependent upon the toxicity achieved after each. Hence, if Grade II toxicity was reached after any single infusion, or at a particular period of time for a steady rate infusion, further doses should be withheld or the steady rate infusion stopped unless toxicity improved. Increasing doses should be administered to groups of patients until approximately 60% of patients showed unacceptable Grade III or IV toxicity in any category. Doses that are 2/3 of this value would be defined as the safe dose.

Physical examination, tumor measurements, and laboratory tests should, of course, be performed before treatment and at intervals of about 3-4 weeks later. Laboratory tests should include complete blood counts, serum creatinine, creatine kinase, electrolytes, urea, nitrogen, SGOT, bilirubin, albumin, and total serum protein.

Clinical responses may be defined by acceptable measure or changes in laboratory values e.g. tumormarkers. For example, a complete response may be defined by the disappearance of all measurable disease for at least a month. Whereas a partial response may be defined by a 50% or greater reduction.

All of the compositions and methods disclosed and claimed herein can be made and executed without undue experimentation in light of the present disclosure.

More specifically, it will be apparent that certain agents which are both chemically and physiologically related may be substituted for the agents described herein while the same or similar results would be achieved.

Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### Administration and dosing

The present invention includes a method of delivery of a pharmaceutically effective amount of the inventive formulation of a low molecular weight compound to an angiogenic vascular target site of a subject in need thereof. A "subject in need thereof" thereby refers to a mammal, e. g. a human.

The route of administration comprises peritoneal, parenteral or topic administration and the formulations are easily administered in a variety of dosage forms such as injectable solutions, drug release capsules and the like.

For use with the present invention the term "pharmacologically effective amount" of a compound administered to a subject in need thereof (which may be any animal with a circulatory system with endothelial cells which undergo angiogenesis) will vary depending on a wide range of factors. For example, it would be necessary to provide substantially larger doses to humans than to smaller animal. The amount of the compound will depend upon the size, age, sex, weight, and condition of the patient as well as the potency of the substance being administered. Having indicated that there is considerable variability in terms of dosing, it is believed that those skilled in the art can, using the present disclosure, readily determine appropriate dosing by first administering extremely small amounts and incrementally increasing the dose until the desired results are obtained. Although the amount of the dose will vary greatly based on factors as described above, in general, the present invention makes it possible to administer substantially smaller amounts of any substance as compared with delivery systems which target the surrounding tissue e. g., target the tumor cells themselves.

The pharmaceutically effective amount of a therapeutic agent as disclosed herein depends on the kind and the type of action of the agent. For the examples mentioned here, it is within the range of about 0.1 to about 20 mg/kg in humans. Typically, for paclitaxel derivatives as well as for camptothecin, doses in the order of 5 mg/kg are applied.

The pharmaceutically effective amount of a diagnostic agent as disclosed herein depends on the type of diagnostic agent. The exact dose depends on the molecular weight of the compound, and on the type and the intensity of the signal to be detected. For the examples as given here (fluorescein as fluorescence dye, gadolinium complexes as MRI markers), the applied dose may range from about 0.1 to 20 mg/kg. Most frequent doses are in the order of about 5 mg/kg.

### References

- 1.: Gregoriadis, G., Liposome Preperation and Related Techiques. Liposome Technology, 1993. 2: p. 123-139.
- 2.: Ceruti, M., et al., Preparation, characterization, cytotoxicity and pharmacokinetics of liposomes containing water-soluble prodrugs of paclitaxel. J Control Release, 2000. 63(1-2): p. 141-53.
- 3.: Burke, T.G., et al., Lipid bilayer partitioning and stability of camptothecin drugs. Biochemistry, 1993. 32(20): p. 5352-64.

## Claims

1. A method of stabilizing a low molecular weight compound from about 200 to about 5000 Da in a liposome, wherein said compound is poorly soluble in a lipid membrane and/or has a low permeability across a lipid membrane, comprising the steps of
(a) providing said compound comprising modifying said compound with a moiety that has a negative net charge, wherein said compound is modified to have a negative net charge,
(b) associating the compound of step a) with a cationic amphiphile having a positive net charge and optionally at least one further anionic and/or neutral amphiphile having a negative and/or neutral net charge and
(c) forming a cationic liposome having a positive zeta potential.

2. The method of claim 1, wherein modifying comprises
(a) covalently linking a negatively charged moiety to said compound, e. g. by an ester, thioester, ether, thioether, amide, amine, carbon-carbon bond or a Schiff Base,
(b) chelating said compound by a negatively charged ligand or
(c) encarcerating said compound within a negatively charged moiety such as a carcerand, calixarene, fullerene, crown or anti-crown ether.

3. The method of any one of the claims 1 or 2, wherein said modifying is reversible.

4. The method of any one of the claims 1 to 3, wherein said compound is a diagnostic agent, a therapeutic agent or a combination thereof.

5. The method of any one of the claims 1 to 4, wherein said compound is selected from diagnostic or imaging agents such as dyes, near-infrared dyes, fluorescent dyes, gold particles, iron oxide particles and other contrast agents including paramagnetic molecules, X-ray attenuating compounds (for CT and X-ray) contrast agents for ultrasound, X-ray emitting isotopes (scintigraphy), and positron-emitting isotopes (PET).

6. The method of any one of the claims 1 to 5, wherein said compound is selected from drugs such as an anti-inflammatory drug, an anti-cancer drug, an enzymatic drug, an antibiotic substance, an antioxidant, a hormone drug, an angiogenesis inhibiting agent, a smooth muscle cell-proliferation/migration inhibitor, a platelet aggregation inhibitor, a release inhibitor for a chemical mediator, and a proliferation/migration inhibitor for vascular endothelium.

7. The method of any one of claims 1 to 6, wherein said cationic amphiphile is selected from lipids, lysolipids or pegylated lipids with a positive net charge.

8. The method of any one of the claims 1 to 7, wherein said cationic amphiphile is selected from quaternary ammonium compounds such as N-(2,3-diacyloxypropyl)-N,N,N-trimethylammonium.

9. The method of any one of claims 1 to 8, wherein said anionic and/or neutral amphiphile is selected from sterols and lipids such as cholesterol, phospholipids, lysolipids, lysophospholipids, sphingolipids or pegylated lipids with a negative or neutral net charge.

10. The method of any one of the claims 1 to 9, wherein the neutral amphiphile is diacylphosphatidylcholine.

11. The method of any one of the claims 1 to 10, wherein the liposome formed in step ( c ) is virtually free of the unmodified compound.

12. A cationic liposome obtainable by a method of any one of the claims 1 to 11.

13. A pharmaceutical composition comprising a pharmaceutically effective amount of the cationic liposome of claim 12, together with a pharmaceutically acceptable carrier, diluent and/or adjuvant.

14. The use of a cationic liposome of claim 12 or a pharmaceutical composition of claim 13 for the preparation of a medicament for diagnosing, preventing and/or treating a condition **characterized by** enhanced angiogenic activity.

15. The use of claim 14, wherein the condition **characterized by** enhanced angiogenic activity is selected from cancer, a chronic inflammatory disease, rheumatoid arthritis, dermatitis, psoriasis and wound healing.

16. The use of claim 15 wherein the active ingredient of the medicament is present in a negatively charged prodrug form.

17. The use of claim 16 wherein the prodrug form is converted to the active drug at a desired target site.

## Patentansprüche

1. Verfahren zum Stabilisieren einer Verbindung mit niedrigem Molekulargewicht von etwa 200 bis etwa 5000 Da in einem Liposom, wobei die Verbindung schlecht in einer Lipidmembran löslich ist und/oder eine geringe Permeabilität durch eine Lipidmembran besitzt, umfassend die Schritte
(a) Bereitstellen der Verbindung umfassend Modifizieren der Verbindung mit einem Rest, der eine negative Nettoladung besitzt, wobei die Verbindung modifiziert wird, dass sie eine negative Nettoladung besitzt,
(b) Assoziieren der Verbindung aus Schritt a) mit einem kationischen Amphiphil, das eine positive Nettoladung besitzt und gegebenenfalls mindestens einem weiteren anionischen und/oder neutralen Amphiphil, das eine negative und/oder neutrale Nettoladung besitzt und
(c) Bilden eines kationischen Liposoms, das ein positives Zeta-Potential besitzt.

2. Verfahren nach Anspruch 1, wobei Modifizieren
(a) kovalentes Verknüpfen eines negativ geladenen Rests an die Verbindung, z. B. durch eine Ester-, Thioester-, Ether-, Thioether-, Amid-, Amin-, Kohlenstoff-Kohlenstoff-Bindung oder eine Schiff-Base,
(b) Chelatisieren der Verbindung durch einen negativ geladenen Liganden oder
(c) Einschließen der Verbindung innerhalb eines negativ geladenen Rests wie etwa einem Carcerand, Calixaren, Fulleren, Kronen- oder Anti-Kronenether
umfasst.

3. Verfahren nach einem der Ansprüche 1 oder 2, wobei das Modifizieren reversibel ist.

4. Verfahren nach einem der Ansprüche 1 bis 3, wobei die Verbindung ein Diagnostikum, ein Therapeutikum oder eine Kombination davon ist.

5. Verfahren nach einem der Ansprüche 1 bis 4, wobei die Verbindung ausgewählt ist aus Diagnostika oder Bildgebungsmitteln wie etwa Farbstoffen, Nah-Infrarot-Farbstoffen, Fluoreszenzfarbstoffen, Goldpartikeln, Eisenoxidpartikeln, und anderen Kontrastmitteln einschließlich paramagnetischer Moleküle, Röntgenstrahlattenuierenden Verbindungen (für CT und Röntgen), Kontrastmittel für Ultraschall, Röntgenstrahl-emittierenden Isotopen (Szintigraphie) und Positron-emittierenden Isotopen (PET).

6. Verfahren nach einem der Ansprüche 1 bis 5, wobei die Verbindung ausgewählt ist aus Arzneimitteln wie etwa einem antiinflammatorischen Arzneimittel, einem Antikrebsmittel, einem enzymatischen Arzneimittel, einer antibiotischen Substanz, einem Antioxidationsmittel, einem Hormonarzneimittel, einem Mittel, das Angiogenese inhibiert, einem Proliferations-/Migrations-Inhibitor glatter Muskelzellen, einem Plättchen-Aggregationsinhibitor, einem Freisetzungsinhibitor für einen chemischen Mediator und einem Proliferations-/Migrations-Inhibitor für vaskuläres Endothel.

7. Verfahren nach einem der Ansprüche 1 bis 6, wobei das kationische Amphiphil ausgewählt ist aus Lipiden, Lysolipiden oder pegylierten Lipiden mit einer positiven Nettoladung.

8. Verfahren nach einem der Ansprüche 1 bis 7, wobei das kationische Amphiphil ausgewählt ist aus quaternären Ammoniumverbindungen wie etwa N-(2,3-diacyloxypropyl)-N,N,N-trimethylammonium.

9. Verfahren nach einem der Ansprüche 1 bis 8, wobei das anionische und/oder neutrale Amphiphil ausgewählt ist aus Sterolen und Lipiden wie etwa Cholesterin, Phospholipiden, Lysolipiden, Lysophospholipiden, Sphingolipiden oder pegylierten Lipiden mit einer negativen oder neutralen Nettoladung.

10. Verfahren nach einem der Ansprüche 1 bis 9, wobei das neutrale Amphiphil Diacylphosphatidylcholin ist.

11. Verfahren nach einem der Ansprüche 1 bis 10, wobei das Liposom, das in Schritt (c) gebildet wird, praktisch frei von der unmodifizierten Verbindung ist.

12. Kationisches Liposom, erhältlich durch ein Verfahren nach einem der Ansprüche 1 bis 11.

13. Pharmazeutische Zusammensetzung, umfassend eine pharmazeutisch wirksame Menge des kationischen Liposoms aus Anspruch 12, zusammen mit einem pharmazeutisch akzeptablen Träger, Verdünnungsmittel und/oder Adjuvans.

14. Verwendung eines kationischen Liposoms nach Anspruch 12 oder einer pharmazeutischen Zusammensetzung nach Anspruch 13 für die Herstellung eines Medikaments zur Diagnose, Prävention und/oder Behandlung eines Zustandes, der durch verstärkte angiogene Aktivität **gekennzeichnet** ist.

15. Verwendung nach Anspruch 14, wobei der Zustand, der durch verstärkte angiogene Aktivität **gekennzeichnet** ist, ausgewählt ist aus Krebs, einer chronischen inflammatorischen Erkrankung, rheumatoider Arthritis, Dermatitis, Psoriasis und Wundheilung.

16. Verwendung nach Anspruch 15, wobei der Wirkstoff des Medikaments in einer negativ geladenen Prodrug-Form vorliegt.

17. Verwendung nach Anspruch 16, wobei die Prodrug-Form an einer gewünschten Zielstelle zum aktiven Arzneimittel umgewandelt wird.

## Revendications

1. Procédé de stabilisation d'un composé de faible masse moléculaire d'environ 200 à environ 5 000 Da dans un liposome, où ledit composé est médiocrement soluble dans une membrane lipidique et/ou a une faible perméabilité à travers une membrane lipidique, comprenant les étapes consistant à
(a) fournir ledit composé comprenant la modification dudit composé avec un fragment qui a une charge nette négative, où ledit composé est modifié pour avoir une charge nette négative,
(b) associer le composé de l'étape (a) avec un amphiphile cationique ayant une charge nette positive et facultativement au moins un amphiphile anionique et/ou neutre supplémentaire ayant une charge nette négative et/ou neutre et
(c) former un liposome cationique ayant un potentiel zêta positif.

2. Procédé selon la revendication 1, dans lequel la modification comprend les sous-étapes consistant à
(a) lier de manière covalente un fragment de charge négative audit composé, par exemple par un ester, thioester, éther, thioéther, amide, amine, une liaison carbone-carbone ou une base de schiff,
(b) chélater ledit composé par un ligand de charge négative ou
(c) incarcérer ledit composé au sein d'un fragment de charge négative tel qu'un carcérand, un calixarène, un fullerène, un éther couronne ou anti-couronne.

3. Procédé selon l'une quelconque des revendications 1 ou 2, dans lequel ladite modification est réversible.

4. Procédé selon l'une quelconque des revendications 1 à 3, dans lequel ledit composé est un agent diagnostique, un agent thérapeutique ou l'une de leurs combinaisons.

5. Procédé selon l'une quelconque des revendications 1 à 4, dans lequel ledit composé est choisi parmi les agents diagnostique ou d'imagerie tel que les teintes, les teintes proche infrarouge, les teintes fluorescentes, les particules d'or, les
particules d'oxyde de fer et d'autres agents de contraste comprenant les molécules paramagnétiques, les composés d'atténuation des rayons X (pour tomodensitométrie et rayons X), les agents de contraste pour ultrasons, les isotopes émettant des rayons X (scintigraphie) et les isotopes émettant des positrons (PET).

6. Procédé selon l'une quelconque des revendications 1 à 5, dans lequel ledit composé est choisi parmi les médicaments tels qu'un médicament anti-inflammatoire, un médicament anticancéreux, un médicament enzymatique, une substance antibiotique, un antioxydant, un médicament hormonal, un agent inhibant l'angiogenèse, un inhibiteur de prolifération/migration des cellules du muscle lisse, un inhibiteur d'agrégation plaquettaire, un inhibiteur de libération pour un médiateur chimique, et un inhibiteur de prolifération/migration pour l'endothélium vasculaire.

7. Procédé selon l'une quelconque des revendications 1 à 6, dans lequel ledit amphiphile cationique est choisi parmi les lipides, lysolipides ou lipides pégylés avec une charge nette positive.

8. Procédé selon l'une quelconque des revendications 1 à 7, dans lequel ledit amphiphile cationique est choisi parmi les composés ammonium quaternaire tels que N-(2,3-diacyloxypropyl)-N,N,N-triméthylammonium.

9. Procédé selon l'une quelconque des revendications 1 à 8, dans lequel ledit amphiphile anionique et/ou neutre est choisi parmi les stérols et lipides tels que cholestérols, phospholipides, lysolipides, lysophospholipides, sphingolipides ou lipides pégylés avec une charge nette négative ou neutre.

10. Procédé selon l'une quelconque des revendications 1 à 9, dans lequel l'amphiphile neutre est la diacylphosphatidylcholine.

11. Procédé selon l'une quelconque des revendications 1 à 10, dans lequel le liposome formé à l'étape (c) est quasiment dépourvu du composé non modifié.

12. Liposome cationique pouvant être obtenu par un procédé selon l'une quelconque des revendications 1 à 11.

13. Composition pharmaceutique comprenant une quantité pharmaceutiquement efficace du liposome cationique de la revendication 12, conjointement avec un support, diluant et/ou adjuvant pharmaceutiquement acceptable.

14. Utilisation d'un liposome cationique de la revendication 12 ou d'une composition pharmaceutique de la revendication 13, pour la préparation d'un médicament destiné à diagnostiquer, prévenir et/ou traiter une affection **caractérisée par** une activité angiogénique accentuée.

15. Utilisation selon la revendication 14, dans laquelle l'affection **caractérisée par** une activité angiogénique accentuée est choisie parmi le cancer, une maladie inflammatoire chronique, la polyarthrite rhumatoïde, la dermatite, le psoriasis et la cicatrisation de plaie.

16. Utilisation selon la revendication 15, dans laquelle l'ingrédient actif du médicament est présent sous une forme de promédicament de charge négative.

17. Utilisation selon la revendication 16, dans laquelle la forme de promédicament est convertie en le médicament actif au niveau d'un site cible souhaité.
